# EUROPEAN PATENT APPLICATION

(11) **EP 1 840 215 A1**
(43) Date of publication of application: **03.10.2007**
(21) Application number: 05822847.9
(22) Date of filing: 27.12.2005
(51) Int. Cl.: C12N 15/09, A61K 45/00, A61P 35/00, A61P 43/00, C12N 9/99, C12Q 1/02, C12Q 1/68

(54) **METHOD OF INHIBITING TELOMERASE ACTIVITY AND INHIBITOR**

(30) Priority: 28.12.2004 JP 2004378834
(71) Applicant: DAIICHI PHARMACEUTICAL CO., LTD., Chuo-ku, Tokyo 103-8234 (JP)
(72) Inventor: WADA, Naoya c/o Daiichi Pharmaceutical Co., Ltd., Tokyo 1348630 (JP); OKAMOTO, Takashi, Tokyo 1340083 (JP); TANIGAKI, Keiji, Nara-shi, Nara 6310061 (JP); DOI, Hirofumi c/o Celestar Lexico-Sciences, Inc., Chiba-shi, Chiba 2618501 (JP); IMAI, Kensaku c/o Celestar Lexico-Sciences, Inc., Chiba-shi, Chiba 2618501 (JP)
(74) Representative: Zwicker, Jörk
(86) International application number: PCT/JP2005/023902
(87) International publication number: WO 2006/070804

(57) **Abstract**

[PROBLEMS] To find out proteins involved in regulation of telomerase activity and to provide a method of inhibiting telomerase activity and a method of enabling the prevention and/or treatment of diseases attributable to the enhanced telomerase activity.

[MEANS FOR SOLVING PROBLEMS] A method of enhancing degradation of telomerase, comprising inhibiting the binding of TERT (telomerase catalytic subunit) to USP21, the binding of NEDD8-conjugated TERT to USP21, or the NEDD8 deconjugation by USP21 from NEDD8-conjugated TERT; a method of inhibiting telomerase activity, comprising using the method of enhancing the degradation; a method of identifying a compound that inhibits the binding or the NEDD8 deconjugation; an agent for inhibiting telomerase activity, comprising the compound; an agent for preventing and/or treating diseases attributable to the enhanced telomerase activity is described and includes, comprising the inhibitory agent; a method of preventing and/or treating the diseases, comprising using the inhibition method or the inhibitory agent a reagent kit.

## Description

### TECHNICAL FIELD

The present invention relates to a method of enhancing degradation of telomerase reverse transcriptase (hereinafter, TERT), comprising inhibiting binding of TERT to ubiquitin specific protease 21 (hereinafter, USP21), and to a method of inhibiting telomerase activity, comprising utilizing the method of enhancing degradation. Further, the present invention relates to a method of enhancing degradation of TERT, comprising inhibiting binding of NEDD8 (neural precursor cell expressed, developmentally down-regulated gene 8)-conjugated TERT to USP21, and to a method of inhibiting telomerase activity, comprising utilizing the method of enhancing degradation.

Further, the present invention relates to a method of enhancing degradation of TERT, comprising inhibiting NEDD8 deconjugation by USP21 from NEDD8-conjugated TERT, and to a method of inhibiting telomerase activity, comprising the method of enhancing degradation.

Furthermore, the present invention relates to a method of identifying a compound that inhibits the binding of TERT to USP21, a compound that inhibits the binding of NEDD8-conjugated TERT to USP21, or a compound that inhibits the NEDD8 deconjugation by USP21 from NEDD8-conjugated TERT.

Further, the present invention relates to an agent for inhibiting telomerase activity, containing the aforementioned compound.

Furthermore, the present invention relates to an agent for preventing and/or treating diseases attributable to increased telomerase activity, containing the aforementioned agent for inhibiting telomerase activity.

Further, the present invention relates to a method of preventing and/or treating diseases attributable to increased telomerase activity, comprising utilizing the aforementioned agent for inhibiting telomerase activity or the aforementioned method of inhibiting telomerase activity.

Furthermore, the present invention relates to a reagent kit, including the following: at least one member selected from the group consisting of USP21, a polynucleotide encoding USP21, a recombinant vector containing the polynucleotide and a transfectant containing the recombinant vector; at lease one member selected from the group consisting of TERT, a polynucleotide encoding TERT, a recombinant vector containing the polynucleotide and a transfectant containing the recombinant vector; and at least one member selected from the group consisting of NEDD8, a polynucleotide encoding NEDD8, a recombinant vector containing the polynucleotide and a transfectant containing the recombinant vector.

### BACKGROUND ART

Telomerase, an enzyme that catalyzes an elongation reaction of the telomere sequence at chromosome ends in eukaryotic cells, is a ribonucleic protein comprising telomerase RNA (hereinafter, TR) that is a template RNA molecule and TERT that is a catalytic subunit (Non-patent Reference 1).

Telomerase endows cells with immortality (unlimited lifespan) by maintaining telomere length. Telomere is made up of a simple repetitive sequence located at the terminal portion of chromosomal DNA in a eukaryotic cell, and contributes to maintaining stability of a chromosome. An ordinary DNA polymerase responsible for DNA replication upon cell division is not capable of replicating DNA completely to the extreme end, resulting in generation of shortened telomere upon cell division. Normal somatic cells repeat cell division for predetermined times and then arrest proliferation to fall in cell senescence or cell death.

Telomerase activity is not detected in normal cells except germ cells, various kinds of stem cells, and peripheral blood lymphocytes. Therefore, most normal cells have a limited lifespan, since telomere length is progressively shortened with cell division and is not maintained.

On the other hand, most immortalized cells such as cancer cells have detectable telomerase activity. In such cells, telomerase allows elongation of telomere shortened upon cell division and maintains telomere length, which is assumed to be one of the mechanisms by which cancer cells acquire unlimited proliferative potential. There are some reports showing that elimination of telomerase activity in cancer cells with antisense RNA resulted in arrest of cell proliferation after repetitive cell division for ten-odd cycles and subsequent induction of cell death. Based upon the reports, it appears that telomerase-dependent maintenance of telomere contributes to immortalization of cancer cells. Moreover, it has been reported that forced expression of dominant negative type telomerase in cancer cells induced elimination of telomerase activity, shortening of telomere length upon cell division, appearance of fused chromosome ends, and cell death.

Activation of telomerase has been reported, such as the activation of telomerase by activation of TERT gene expression, and the activation of telomerase via phosphorylation of TERT. With regard to the former, most normal cells have been found to express TR gene, and not TERT gene, while many cancer cells are found to express both TR and TERT genes. Therefore, cells with detectable telomerase activity, such as cancer cells, are assumed to have a certain mechanism to activate expression of TERT gene. With regard to the latter, it has been reported that telomerase activity in nuclear extracts from cancer cells was reduced by dephosphorylation treatment of the nuclear extracts, suggesting possible involvement of phophorylation in the activation of telomerase (Non-patent Reference 2). Moreover, it has been reported that telomerase activity in nuclear extracts from cancer cells was enhanced by bringing the nuclear extracts into reaction with Akt or Protein kinase C (PKC), suggesting possible involvement of these kinases in the activation of telomerase (Non-patent References 3 and 4).

Furthermore, there has been a report published after the priority date of the present application, which shows ubiquitination of TERT, degradation of TERT by proteasome, MKRN1 as an E3 ligase promoting ubiquitination of TERT, and regulation of telomerase activity by MKRN1 (Non-patent Reference 11, April 1, 2005).

However, a specific enzyme that deubiquitinates the ubiquitinated TERT has never been reported so far. Furthermore, there are no reports on regulation of telomerase activity by other post-translational modifications of TERT such as acetylation, glycosylation, and NEDD8 conjugation.

NEDD8 is a ubiquitin-like protein having approximately 80% homology with ubiquitin at amino acid level and consists of 81 amino acid residues. In the presence of ubiquitin ligase, ubiquitin covalently conjugates to a lysine residue on a target protein. Subsequently, the other ubiquitin covalently conjugates to a lysine residue on the ubiquitin that conjugated to the lysine residue on the target protein. Thus, a plurality of ubiquitins are conjugated to a protein in a chain-like form. The phrase "protein ubiquitination" refers to protein modification with ubiquitin via covalent conjugation of one or more ubiquitins to one molecule of protein. In addition, the phrase "ubiquitinated protein" refers to a protein modified with one or more ubiquitins. A ubiquitinated protein undergoes proteasome-dependent degradation. Accordingly, ubiquitination is assumed to be involved in regulation of protein stability (Non-patent Reference 5).

Similar to ubiquitin, NEDD8 covalently conjugates to a lysine residue on a protein in post-translational modification of protein. The phrase "NEDD8 conjugation" refers to covalent conjugation of NEDD8 to protein. Only a cullin family protein, which is one of the subunits of a ubiquitin ligase complex, has been reported as a protein that undergoes NEDD8 conjugation. In addition, it has been suggested that there exists a mechanism of regulation of ubiquitin-ligase activity through NEDD8 conjugation to cullin (Non-patent References 5 and 6).

Significant homology of NEDD8 with ubiquitin at the level of both primary structure and tertiary structure (Non-patent Reference 5) suggests that NEDD8 conjugation to proteins may work as a signal for proteasome-dependent protein degradation (Non-patent Reference 5), similar to protein ubiquitination working as a signal for protein degradation. However, there has been no report indicating the presence of a NEDD8 conjugation-mediated and proteasome-dependent system for protein degradation.

Proteasome is a protease complex. 20S proteasome, 26S proteasome and football-like proteasome are known. 26S proteasome is involved in selective and energy-dependent degradation of ubiquitinated proteins. 26S proteasome is an ATP-dependent protease complex with sedimentation coefficient of 26S, which is composed of an ATP-independent protease complex named 20S proteasome with two PA700 molecules bound thereto. 20S proteasome has a hollow structure that is composed of α rings consisting of seven α subunits each and β rings consisting of seven β subunits each, where the rings build up in order such as α, β, β, α to form a cylinder shape. PA700 binds to the top of and the bottom of 20S proteasome to form 26S proteasome. PA700, which is an internal activator capable of activating proteasome that is normaly inactive, is a multi-component complex having a ubiquitin-recognition subunit as a component. A ubiquitinated protein is recognized by PA700 that is a component of 26S proteasome, and is recruited into the proteasome. In the proteasome, the ubiquitinated protein degenerates and subsequently releases ubiquitins, and is finally degraded by β subunits.

NUB1 (NEDD8 ultimate buster-1) is known to be a protein that binds to NEDD8 (Non-patent Reference 7). Further, it has been reported that NUB 1 binds to S5a, a component protein of 19S subunit of 26S proteasome, and that the over-expression thereof leads to a greater co-precipitation of NEDD8-conjugated proteins with S5a. (Non-patent Reference 8). These reports suggest that NUB1 recognizes the NEDD8 moiety of NEDD8-conjugated proteins, and thereby works as a recruiter protein in the transportation of the NEDD8-conjugated proteins to 26S proteasome, resulting in the degradation of the NEDD8-conjugated proteins in the 26S proteasome.

USP21 is a cysteine protease, which has been reported to have activity as an enzyme for deubiquitination to remove ubiquitins from ubiquitinated proteins, and for NEDD8 deconjugation to remove NEDD8 from NEDD8-conjugated proteins (Non-patent Reference 9). Further, similar to USP21, DEN1 (human deneddylase 1) has been reported to have activity of deconjugating NEDD8 from NEDD8-conjugated cullin (Non-patent Reference 10).

While USP21 has been reported to show NEDD8 deconjugation activity unspecifically against proteins, proteins which have undergone NEDD8 deconjugation have not been identified yet. Further, there are no reports indicating relationships between NEDD8 or USP21 and telomerase, and no reports of proteasome-system-dependent telomerase degradation.

Patent Reference 1: "International Publication No. WO 01/67299 pamphlet".
Non-patent Reference 1: Kelleher C. et al., "TRENDS in Biochemical Sciences", 2002, Vol.27, p.572-579.
Non-patent Reference 2: "The Journal of Biological Chemistry", 1997, Vol.272, p.16729-16732.
Non-patent Reference 3: "The Journal of Biological Chemistry", 1999, Vol.274, p.13085-13090.
Non-patent Reference 4: "The Journal of Biological Chemistry", 1998, Vol.273, p.33436-33442.
Non-patent Reference 5: "Gene", 2000, Vol. 248, p.1-14.
Non-patent Reference 6: "Jikken Igaku (Experimental Medicine) ", 2004, Vol. 22, No. 2, p. 69-75. Non-patent Reference 7: "The Journal of Biological Chemistry", 2001, Vol. 276, No. 23, p.20603-20609.
Non-patent Reference 8: "The Journal of Biological Chemistry", 2001, Vol. 276, No. 49, p.46655-46660.
Non-patent Reference 9: "The Journal of Biological Chemistry", 2000, Vol. 75, p.14212-14216.
Non-patent Reference 10: "The Journal of Biological Chemistry", 2003, Vol. 278, No. 31, p.28882-28891.
Non-patent Reference 11: "Genes & Development", 2005 Vol. 19, p.776-781.

### DISCLOSURE OF THE INVENTION

### [PROBLEM TO BE SOLVED BY THE INVENTION]

An object of the present invention is to identify and to provide a protein involved in regulation of telomerase activity. Further the object of the present invention includes providing a means of inhibiting telomerase activity. Furthermore, the object of the present invention includes providing a means of allowing for prevention and/or treatment of diseases attributable to increased telomerase activity, for example, cancer diseases.

### [MEANS FOR SOLVING THE PROBLEM]

The present inventors have intensively concentrated efforts to solve the aforementioned objects, and predicted *in-silico* that TERT, which is a catalytic subunit of telomerase, may interact with both NEDD8, a ubiquitin-like protein, and USP21, a cysteine protease. Then, the present inventors demonstrated that TERT binds to both NEDD8 and USP21. Further, the present inventors revealed the following facts: TERT undergoes NEDD8 conjugation; NEDD8-conjugated TERT undergoes USP21-mediated NEDD8 deconjugation; proteasome-dependent degradation of NEDD8-conjugated TERT results in proteasome-dependent degradation of TERT; TERT is stabilized by USP21; and nuclear telomerase activity is increased by USP21 in a manner dependent on cysteine protease activity of USP21. These experimental results led the present inventors to discover that there is a proteasome-dependent regulatory system for TERT degradation via NEDD8 conjugation to TERT and NEDD8 deconjugation by USP21 from NEDD8-conjugated TERT, and that the system regulates nuclear telomerase activity. The present invention has been thus achieved.

In various embodiments, the present invention relates to a method of enhancing degradation of TERT, comprising inhibiting binding of NEDD8-conjugated TERT to USP21.

The present invention also relates to a method of enhancing degradation of TERT, comprising inhibiting NEDD8 deconjugation by USP21 from NEDD8-conjugated TERT.

The present invention further relates to a method of inhibiting telomerase activity, comprising utilizing the aforementioned method of enhancing degradation of TERT.

The present invention still further relates to a method of identifying a compound that inhibits binding of USP21 to TERT, comprising contacting a compound to USP21 and/or TERT under conditions where the interaction of the compound with USP21 and/or TERT is allowed, introducing a system using a signal and/or a marker that is generated by the binding of USP21 to TERT, and detecting the presence, absence or change of the signal and/or the marker to determine whether the compound inhibits the binding of USP21 to TERT.

The present invention also relates to a method of identifying a compound that inhibits binding of USP21 to NEDD8-conjugated TERT, comprising contacting a compound to USP21 and/or NEDD8-conjugated TERT under conditions where the interaction of the compound with USP21 and/or NEDD8-conjugated TERT are allowed, introducing a system using a signal and/or a marker that is generated by the binding of USP21 to NEDD8-conjugated TERT, and detecting the presence, absence or change of the signal and/or the marker to determine whether the compound inhibits the binding of USP21 to NEDD8-conjugated TERT.

The present invention further relates to a method of identifying a compound that inhibits NEDD8 deconjugation by USP21 from NEDD8-conjugated TERT, comprising contacting a compound to USP21 and/or NEDD8-conjugated TERT under conditions where the interaction of the compound with USP21 and/or NEDD8-conjugated TERT is allowed, introducing a system using a signal and/or a marker that is generated by the NEDD8 deconjugation by USP21 from NEDD8-conjugated TERT, and detecting the presence, absence or change of the signal and/or the marker to determine whether the compound inhibits the NEDD8 deconjugation by USP21 from NEDD8-conjugated TERT.

The present invention still further relates to an agent for inhibiting telomerase activity, containing a compound that inhibits binding of USP21 to NEDD8-conjugated TERT.

The present invention also relates to an agent for inhibiting telomerase activity, containing a compound that inhibits NEDD8 deconjugation by USP21 from NEDD8-conjugated TERT.

The present invention further relates to an agent for preventing and/or treating diseases attributable to increased telomerase activity, containing the aforementioned agent for inhibiting telomerase activity.

The present invention still further relates to an anti-cancer agent, containing the aforementioned agent for inhibiting telomerase activity.

The present invention also relates to a method of preventing and/or treating diseases attributable to increased telomerase activity, comprising utilizing the aforementioned method of inhibiting telomerase activity.

The present invention further relates to a method of preventing and/or treating cancer diseases, comprising utilizing the aforementioned method of inhibiting telomerase activity.

The present invention still further relates to a method of preventing and/or treating diseases attributable to increased telomerase activity, comprising utilizing the aforementioned agent for inhibiting telomerase activity.

The present invention also relates to a method of preventing and/or treating cancer diseases, comprising utilizing the aforementioned agent for inhibiting telomerase activity.

The present invention further relates to a reagent kit, comprising at least one member selected from the group consisting of USP21, a polynucleotide encoding USP21, a vector containing the polynucleotide, and a transformant containing the vector; and at least one member selected from the group consisting of TERT, a polynucleotide encoding TERT, a vector containing the polynucleotide, and a transformant containing the vector; and at least one member selected from the group consisting of NEDD8, a polynucleotide encoding NEDD8, a vector containing the polynucleotide, and a transformant containing the vector.

### [ADVANTAGE OF THE INVENTION]

In the present invention, it was predicted, *in-silico,* that TERT may interact with both USP21 and NEDD8, and then the predicted possibility was experimentally demonstrated. As a result, the following facts were found: TERT binds directly to USP21; TERT undergoes NEDD8 conjugation; NEDD8-conjugated TERT undergoes USP21-mediated NEDD8 deconjugation; proteasome-dependent degradation of NEDD8-conjugated TERT results in proteasome-dependent degradation of TERT; and TERT is stabilized by USP21.

These experimental results led to the findings that degradation (namely, stability) of TERT is regulated by NEDD8 conjugation to TERT and NEDD8 deconjugation by USP21 from NEDD8-conjugated TERT. Furthermore, USP21 was found to increase nuclear telomerase activity in a manner dependent on its cysteine protease activity. Thus, there was found a proteasome-dependent regulatory system for TERT degradation via NEDD8 conjugation to TERT and NEDD8 deconjugation by USP21 from NEDD8-conjugated TERT, which regulates nuclear telomerase activity.

Accordingly, proteasome-dependent degradation, for example 26S proteasome-dependent degradation, of NEDD8-conjugated TERT can be enhanced by inhibiting USP21-mediated NEDD8 deconjugation from NEDD8-conjugated TERT. The enhanced proteasome-dependent degradation of NEDD8-conjugated TERT leads to enhanced degradation of TERT, resulting in a reduced amount of intracellular TERT. Since TERT is a catalytic subunit of telomerase, a reduced amount of TERT leads to inhibition of telomerase activity.

The inhibition of USP21-mediated NEDD8 deconjugation from NEDD8-conjugated TERT can be achieved, for example, by inhibiting binding of USP21 to NEDD8-conjugated TERT. Accordingly, proteasome-dependent degradation of NEDD8-conjugated TERT can be enhanced by inhibiting the binding of USP21 to NEDD8-conjugated TERT, thereby leading to enhancement of TERT degradation which results in inhibition of telomerase activity.

Thus, the present invention makes it possible to inhibit telomerase activity. Therefore, the present invention allows carrying out prevention and/or treatment of diseases attributable to increased telomerase activity. Telomerase endows cells with immortality (unlimited lifespan), and its activity is detected in immortalized cells such as cancer cells. The inhibition of telomerase activity according to the present invention facilitates limiting the lifespan of cancer cells and destabilizing chromosome, resulting in induction of cell death. Accordingly, the present invention allows carrying out prevention and/or treatment of, for example, cancer diseases.

### BRIEF DESCRIPTION OF THE DRAWINGS

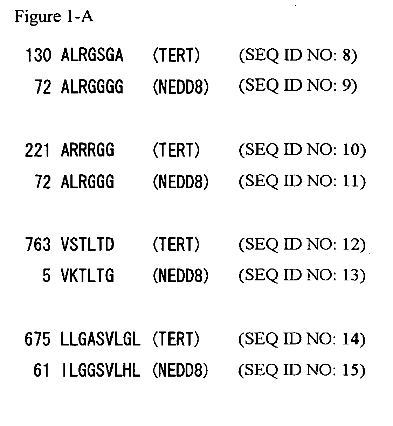

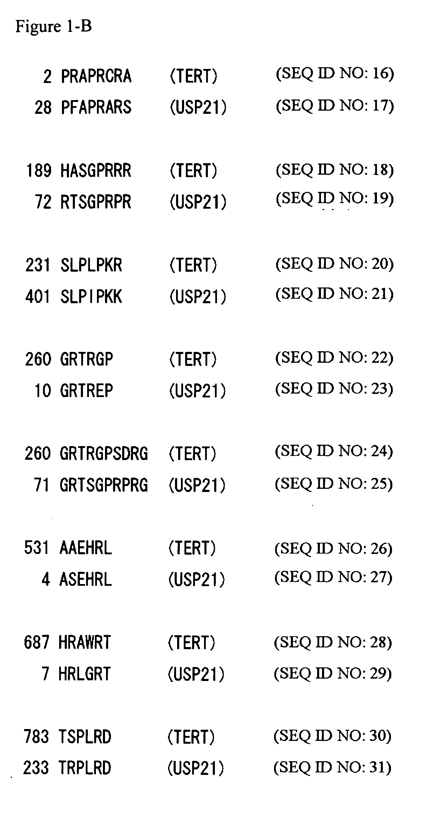

Figure 1-A shows results *of in silico* prediction that TERT interacts with NEDD8. The region that exhibited a high score as a result of local alignment between TERT and NEDD8 is shown. The amino acid sequences are represented in the single-letter code. The number in the figure indicates the position of the N-terminal amino acid of each region shown in the figure in the amino acid sequence of TERT or NEDD8 (Example 1).
Figure 1-B shows results of *in silico* prediction that TERT interacts with USP21. The region that exhibited a high score as a result of local alignment between TERT and USP21 is shown. The amino acid sequences are represented in the single-letter code. The number in the figure indicates the position of the N-terminal amino acid of each region shown in the figure in the amino acid sequence of TERT or USP21. (Example 1)

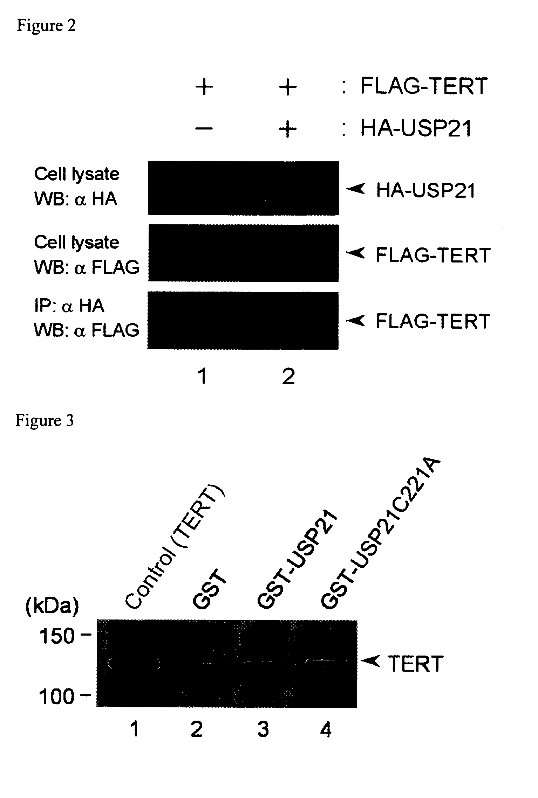

Figure 2 shows that co-precipitation of N-terminal FLAG-tagged TERT (FLAG-TERT) with N-terminal HA-tagged USP21 (HA-USP21) was detected in a cell lysate prepared from cultured cells in which FLAG-TERT was transiently co-expressed with HA-USP21, by immunoprecipitation (IP) of HA-USP21 with anti-HA antibodies (αHA) (lane 2, bottom panel). The co-precipitation of FLAG-TERT was determined by Western blotting (WB) using anti-FLAG antibodies (αFLAG). In contrast, co-precipitation of FLAG-TERT by using anti-HA antibodies was not detected when using cells in which only FLAG-TERT was expressed (lane 1, bottom panel). The expression of HA-USP21 or FLAG-TERT in cultured cells was determined by Western blotting using anti-HA antibodies or anti-FLAG antibodies, respectively (lanes 1 and 2, top panel and the middle panel). (Example 2)
Figure 3 shows that binding of TERT to both N-terminal glutathione S-transferase (GST)-fused USP21 (GST-USP21) and N-terminal GST-fused USP21 inactive mutant (GST-USP21C221A) was detected in *in vitro* binding assay using a GST pull down method (lanes 3 and 4, respectively). In contrast, binding of TERT to GST was not observed (lane 2). (Example 2)

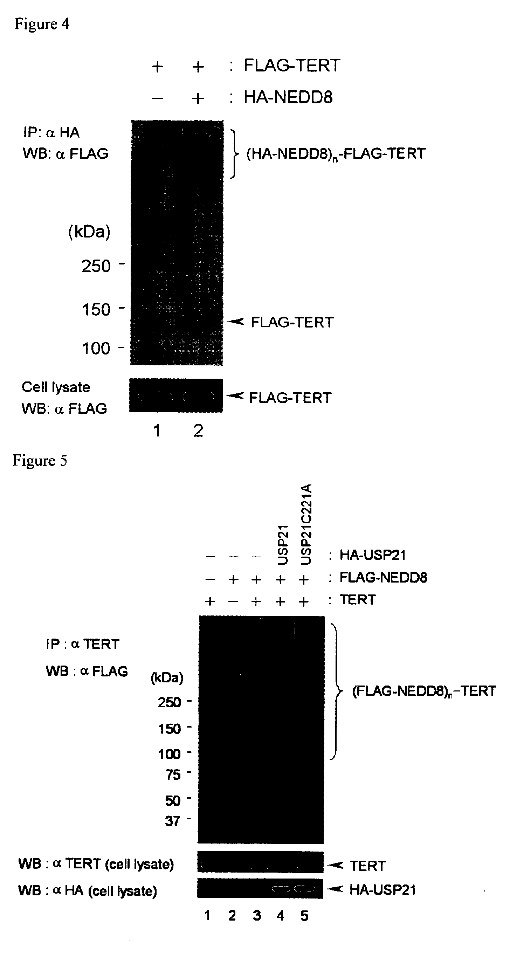

Figure 4 shows that co-precipitation of N-terminal FLAG-tagged TERT (FLAG-TERT) with N-terminal HA-tagged NEDD8 (HA-NEDD8) was detected in a cell lysate prepared from cultured cells in which FLAG-TERT was transiently co-expressed with HA-NEDD8, by immunoprecipitation (IP) of HA-NEDD8 with anti-HA antibodies (αHA) (lane 2, upper panel). The co-precipitation of FLAG-TERT was determined by Western blotting (WB) using anti-FLAG antibodies (αFLAG). A signal was detected with a smear pattern in the high molecular weight area which is assumed to be TERT being conjugated with one or more HA-NEDD8 molecules ((HA-NEDD8)n-FLAG-TERT). In contrast, co-precipitation of FLAG-TERT by using anti-HA antibodies and a signal with a smear pattern in the high molecular weight area were not detected when using cells in which only FLAG-TERT was expressed (lane 1, upper panel). The amount of FLAG-TERT was almost the same in the cell lysate prepared from cells in which only FLAG-TERT was expressed and in the cell lysate prepared from cells in which FLAG-TERT and HA-NEDD8 were co-expressed (lanes 1 and 2, respectively, lower panel). (Example 2)
Figure 5 shows that a signal was detected with a smear pattern in the high molecular weight area which is assumed to be TERT being conjugated with one or more FLAG-NEDD8 molecules ((FLAG-NEDD8)n-FLAG-TERT) in a cell lysate prepared from cultured cells in which FLAG-TERT was transiently co-expressed with HA-NEDD8 (lane 3, top panel). The figure also shows that the signal was significantly reduced by co-expression of N-terminal HA-tagged USP21 (HA-USP-21) (lane 4, top panel). Such a signal was not observed in a cell lysate prepared from cultured cells in which TERT or FLAG-NEDD8 was transiently expressed individually (lanes 1 and 2, respectively, top panel). In addition, reduction of the signal was not observed in a case of co-expressing N-terminal HA-tagged USP21 inactive mutant (HA-USP21C221A) (lane 5, top panel). The amount of TERT was almost the same in each cell lysate prepared from the cells expressing TERT only, the cells co-expressing TERT together with FLAG-NEDD8, the cells co-expressing TERT together with FLAG-NEDD8 and HA-USP21, and the cells co-expressing TERT, FLAG-NEDD8 and HA-USP21C221A (lanes 1, 3, 4 and 5, respectively, middle panel). Further, the expression of HA-USP21 or HA-USP21C221A in the cell lysates was confirmed (lanes 4 and 5, bottom panel). (FLAG-NEDD8)n-FLAG-TERT was determined by immunoprecipitation (IP) with anti-TERT antibodies (αTERT) followed by Western blotting (WB) with anti-FLAG antibodies (αFLAG). The detection of TERT was carried out by Western blotting with αTERT. The detection of HA-USP21 and HA-USP21C221A was carried out by Western blotting with anti-HA antibodies (αHA). (Example 2)

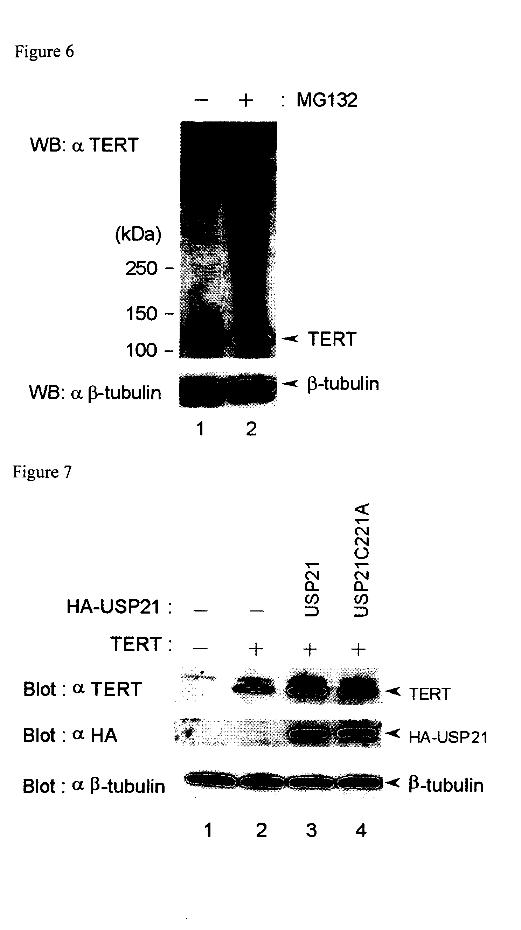

Figure 6 shows that the amount of TERT was increased in a cell lysate prepared from cells treated with a proteasome inhibitor MG132 (lane 2, upper panel) in comparison to a cell lysate prepared from cells not treated with MG132 (lane 2, upper panel). There was no difference in the amount of β-tubulin, an internal protein (lanes 1 and 2, lower panel). Both proteins, TERT and β-tubulin, were determined by Western blotting (WB) with anti-TERT antibodies (αTERT) and anti-β-tubulin antibodies (αβ-tubulin), respectively. (Example 4)
Figure 7 shows that the amount of TERT was increased in a cell lysate prepared from cells in which N-terminal HA-tagged USP21 (HA-USP21) was co-expressed with TERT (lane 3, top panel) in comparison to a cell lysate prepared from cells in which only TERT was expressed or cells in which N-terminal HA-tagged USP21 inactive mutant (HA-USP21C221A) was co-expressed with TERT (lanes 2 and 4, respectively, top panel). There was no difference in the amount of β-tubulin, an internal protein (lanes 1 and 4, bottom panel). The amount of USP21 or USP21C221A in each cell lysate was almost the same (lanes 3 and 4, middle panel). Both proteins, TERT and β-tubulin, were determined by Western blotting (Blot) with anti-TERT antibodies (αTERT) and anti-β-tubulin antibodies (αβ-tubulin), respectively. HA-UP 21 and HA-USP21C221A were determined by Western blotting with anti-HA antibodies (αHA). (Example 5)

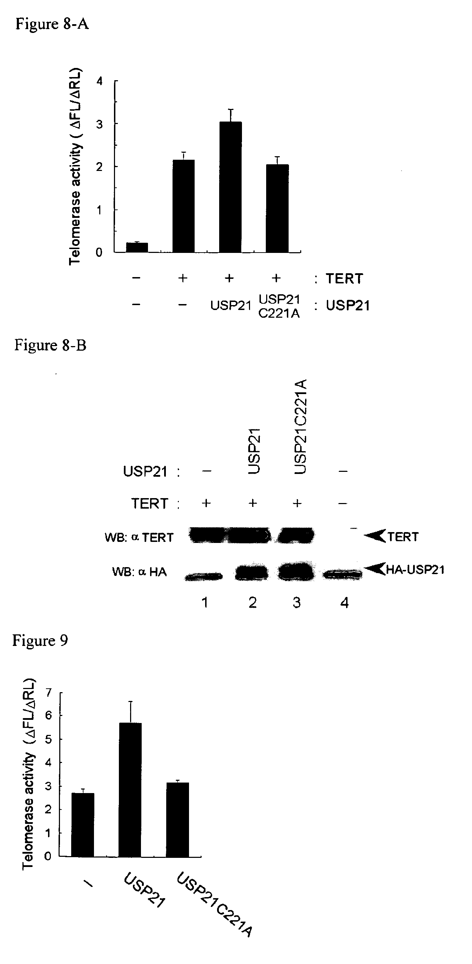

Figure 8-A shows that nuclear telomerase activity in SaOS2 cells having low nuclear telomerase activity was increased by over-expression of N-terminal HA-tagged USP21 (HA-USP21) together with TERT in comparison to expression of TERT only. The increase of nuclear telomerase activity achieved by over-expression of HA-USP21 was not observed by over-expression of N-terminal HA-tagged USP21 inactive mutant (HA-USP21C221A). (Example 6)
Figure 8-B shows that the amount of TERT in SaOS2 cells having low nuclear telomerase activity was increased by over-expression of N-terminal HA-tagged USP21 (HA-USP21) together with TERT (lane 1, upper panel) in comparison to expression of TERT only (lane 2, upper panel). The increase of the amount of TERT was not observed in a cell lysate prepared from cells in which N-terminal HA-tagged USP21 inactive mutant (HA-USP21C221A) was over-expressed together with TERT (lane 3, upper panel). The expression of HA-USP21 and HA-USP21C221A in the cells was confirmed by Western blotting (WB) (lanes 2 and 3, respectively, lower panel). TERT was determined by Western blotting (WB) with anti-TERT antibodies (αTERT). HA-USP21 and HA-USP21C221A was determined by Western blotting (WB) with anti-HA antibodies (αHA). (Example 6)
Figure 9 shows that nuclear telomerase activity was increased by over-expression of N-terminal HA-tagged USP21 (HA-USP21). The increase of telomerase activity was not observed by over-expression of N-terminal HA-tagged USP21 inactive mutant (HA-USP21C221A).

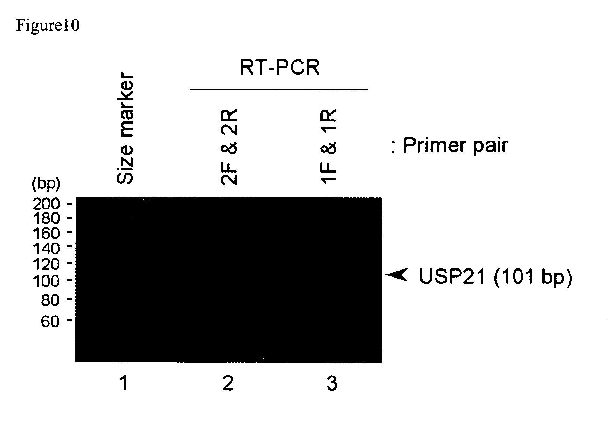

Figure 10 shows results of detection of human USP21 gene expression in HeLa cells (lanes 2 and 3) by using RT-PCT. cDNA fragments of human USP21 gene was determined by ethidium bromide staining. RT-PCR was carried out using each of two sets of primer pairs, 1F and 1R, and 2F and 2R. The lane 1 shows size markers. (Example 8)

### DETAILED DESCRIPTION OF THE INVENTION

Embodiments of the present invention are explained in further detail below.
In the present specification, the term "protein" may be used as a generic term which includes the following: an isolated or a synthetic full-length protein; an isolated or a synthetic full-length polypeptide; and an isolated or a synthetic full-length oligopeptide. A protein, a polypeptide, or an oligopeptide used herein comprises two or more amino acids that are bound to each other by peptide bond or modified peptide bond. Hereinafter, an amino acid may be represented by a single letter or by three letters.

In the present invention, it was predicted, *in-silico,* that TERT may interact with both USP21 and NEDD8, and then the possibility was experimentally demonstrated. As a result, the following facts were found: TERT binds directly to USP21; TERT undergoes NEDD8 conjugation; NEDD8-conjugated TERT undergoes USP21-mediated NEDD8 deconjugation; proteasome-dependent degradation of NEDD8-conjugated TERT results in proteasome-dependent degradation of TERT; and TERT is stabilized by USP21.

These experimental results led to the findings that degradation (namely, stability) of TERT is regulated by NEDD8 conjugation to TERT and NEDD8 deconjugation by USP21 from NEDD8-conjugated TERT. Furthermore, USP21 was found to increase nuclear telomerase activity in a manner dependent on its cysteine protease activity.

Meanwhile, since NEDD8 has significant homology with ubiquitin at the level of both primary structure and tertiary structure (Non-patent Reference 5), it has been suggested that NEDD8 conjugation to proteins works as a signal for proteasome-dependent protein degradation, similar to protein ubiquitination (Non-patent Reference 5). In addition, MKRN1-induced ubiquitination of TERT and subsequent degradation of ubiquitinated TERT by proteasome have been reported (Non-patent reference 11). The present invention demonstrated that proteasome-dependent degradation of NEDD8-conjugated TERT resulted in proteasome-dependent degradation of TERT. From the experimental results shown in the present application, it can be reasoned that NEDD8-conjugated TERT is degraded by proteasome.

The evidence described above led to the findings that there is a proteasome-dependent regulatory system for TERT degradation via NEDD8 conjugation to TERT and NEDD8 deconjugation by USP21 from NEDD8-conjugated TERT, and that the system regulates telomerase activity in a cell. The present inventors believe that proteasome is involved in the regulatory system for TERT degradation. Thus, the present inventors believe that NEDD8-conjugated TERT is degraded by proteasome. Therefore, the present inventors believe that NEDD8 conjugation to TERT leads to degradation of TERT and that the degradation of TERT is inhibited by NEDD8 deconjugation by USP21 from NEDD8-conjugated TERT.

Accordingly, proteasome-dependent degradation of NEDD8-conjugated TERT can be enhanced by inhibiting USP21-mediated NEDD8 deconjugation from NEDD8-conjugated TERT. Further, inhibiting the USP21-mediated NEDD8 deconjugation from NEDD8-conjugated TERT can be carried out by inhibiting binding of NEDD8-conjugated TERT to USP21, and consequently enhancing the proteasome-dependent degradation of NEDD8-conjugated TERT.

Since TERT is a catalytic subunit of telomerase, reducing the amount of TERT by enhancing degradation of NEDD8-conjugated TERT results in inhibition of telomerase activity. Therefore, inhibiting telomerase activity can be carried out by enhancing the degradation of NEDD8-conjugated TERT.

An aspect of the present invention achieved based on these findings relates to a method of enhancing degradation of TERT which comprises inhibiting NEDD8 deconjugation by USP21 from NEDD8-conjugated TERT, and a method of inhibiting telomerase activity which comprises utilizing the method of enhancing degradation. Further, another aspect of the present invention relates to a method of enhancing degradation of TERT which comprises inhibiting binding of NEDD8-cojugated TERT to USP21, and a method of inhibiting telomerase activity which comprises utilizing the method of enhancing degradation. The inhibition of the binding and/or inhibition of the NEDD8 deconjugation enhances proteasome-dependent degradation, for example, 26S proteasome-dependent degradation, of NEDD8-conjugated TERT, and consequently enhances degradation of TERT, which results in reduction of the amount of TERT in cells and inhibition of telomerase activity.

"TERT", a protein component of telomerase, is a catalytic subunit providing enzyme activity of telomerase. TERT gene and a protein encoded by the gene can be, for example, a polynucleotide and a protein originated in humans which are respectively shown by SEQ ID NO: 1 and SEQ ID NO: 2 in the sequence listing. Telomerase is an enzyme that catalyzes the elongation reaction of telomere sequence at chromosome ends in eukaryotic cells to maintain telomere length, and thereby endows cells with immortality (unlimited lifespan).

"NEDD8" is a ubiquitin-like protein having homology with ubiquitin of approximately 80% at amino acid level and consists of 81 amino acid residues. NEDD8 gene and a protein encoded by the gene can be, for example, a polynucleotide and a protein originated in humans which are respectively shown by SEQ ID NO: 3 and SEQ ID NO: 4 in the sequence listing. It has been identified that ubiquitin is ubiquitinated on lysine residue at position 48 in its amino acid sequence. NEDD8 also has a lysine residue at position 48 in its amino acid sequence. Further, the reaction mechanism of NEDD8 conjugation is considered to be similar to that of ubiquitination. These facts suggests that NEDD8 covalently conjugates to another NEDD8 on lysine residue at position 48 in the amino acid sequence by isopeptide bond formation, resulting in conjugation of a plurality of NEDD8 molecules to a protein in a chain-like form (Non-patent Reference 5).

The phrase "NEDD8 conjugation" refers to protein modification with NEDD8 via covalent conjugation of one or more NEDD8 molecules to one molecule of protein. NEDD8 is assumed to covalently conjugate to a lysine residue in a target protein. Similar to protein ubiquitination, NEDD8 covalently conjugates to the lysine residue of the other NEDD8 that conjugated to the lysine residue of a target protein, resulting in conjugation of a plurality of NEDD8 molecules to a protein in a chain-like form

The phrase "NEDD8-conjugated TERT" refers to TERT that was conjugated with NEDD8, that is to say, TERT that was modified with NEDD8 via covalent conjugation of one or more NEDD8 molecules to one molecule of TERT.

"Proteasome" is preferably 26S proteasome. 26S proteasome is a protease complex participating in selective and energy-dependent degradation of ubiquitinated proteins. NUB1, which binds to a subunit component of 26S proteasome, has been reported to bind to NEDD8 (Non-patent Reference 7). Further, NUB1 has been reported to bind to S5a, a component protein of 19S subunit of 26S proteasome, and the over-expression thereof has been reported to lead to a greater co-precipitation of NEDD8-conjugated proteins with S5a. (Non-patent Reference 8). Thus, it is suggested that NUB1 recognizes NEDD8 moiety of NEDD8-conjugated proteins, and thereby works as a recruiter protein in transportation of the NEDD8-conjugated proteins to 26S proteasome, resulting in degradation of the NEDD8-conjugated proteins in the 26S proteasome. Accordingly, "proteasome" is, for example, 26S proteasome. However, proteasome that participates in a system for proteasome-dependent degradation of NEDD8-conjugated TERT is not limited to 26S proteasome, and can be any proteasome as long as it participates in the system for degradation of NEDD8-conjugated TERT.

The phrase "proteasome-dependent degradation of NEDD8-conjugated TERT" refers to a series of reactions in which NEDD8-conjugated TERT is transported to proteasome by the action of recruiter proteins such as NUB1, and subsequently suffers degradation in the proteasome.

The present invention revealed the binding of USP21 to TERT (refer to Example 2). In addition, the present invention demonstrated the NEDD8 deconjugation by USP21 from NEDD8-conjugated TERT (refer to Example 3).

These findings show that USP21 binds to TERT, and that TERT then undergoes NEDD8 conjugation by a NEDD8 conjugation system in cells, resulting in generation of NEDD8-conjugated TERT that subsequently undergoes NEDD8 deconjugation by the USP21.

The phrase "NEDD8 deconjugation by USP21 from NEDD8-conjugated TERT" refers to a series of reactions in which USP21 binds to TERT, and then the TERT undergoes NEDD8 conjugation by a NEDD8 conjugation system in cells resulting in generation of NEDD8-conjugated TERT, and subsequently from which NEDD8 is released in a manner dependent on cystein protease activity of the USP21.

Given these findings, the present inventors believe that NEDD8 deconjugation by USP21 from NEDD8-conjugated TERT can be inhibited by inhibiting the binding of USP21 to TERT.

Thus, further aspects of the present invention relate to a method of enhancing degradation of TERT which comprises inhibiting the binding of USP21 to TERT and a method of inhibiting telomerase activity which comprises utilizing the method of enhancing degradation. The inhibition of the binding allows enhanced degradation of NEDD8-conjugated TERT, resulting in enhanced degradation of TERT that leads to reduced TERT in cells and consequent inhibition of telomerase activity.

"USP21" is a cysteine protease and has been reported to have activity as an enzyme for deubiquitination to remove ubiquitins from ubiquitinated proteins and for NEDD8 deconjugation to remove NEDD8 from NEDD8-conjugated proteins (Non-patent Reference 9). USP21 gene and a protein encoded by the gene can be, for example, a polynucleotide and a protein originated in humans which are respectively shown by SEQ ID NO: 5 and SEQ ID NO: 6 in the sequence listing.

Detection of NEDD8 deconjugation by USP21 from NEDD8-conjugated TERT can be carried out, for example, by using eukaryotic cells or cultured cell lines in which expression of TERT or telomerase activity is found, making the cells or cell lines transiently express USP21, and measuring the amount of NEDD8-conjugated TERT and/or the amount of TERT in cell lysates of the cells or cell lines (refer to Example3). The amount of NEDD8-conjugated TERT and the amount of TERT can be measured by, for example, Western blotting and the like that are well-known in the art. The telomerase activity can be measured by a well-known method of measuring telomerase activity such as a TRAP method. NEDD8 conjugation to TERT in cells is performed by an internal NEDD8 conjugation system. Where the amount of NEDD8-conjugated TERT in a cell lysate is diminished or eliminated, or where the amount of TERT in a cell lysate is increased or generated, after carrying out NEDD8 deconjugation, it can be determined that NEDD8-conjugated TERT underwent NEDD8 deconjugation by USP21. Regarding cells for use, cells that are made to transiently express NEDD8-conjugated TERT can also be used. The expression of NEDD8-conjugated TERT in cells can be carried out using an adequate vector containing a polynucleotide encoding TERT and an adequate vector containing a polynucleotide encoding NEDD8, by transfecting them into cells by conventional genetic engineering techniques.

Detection of NEDD8 deconjugation by USP21 from NEDD8-conjugated TERT can be carried out, for example, by using eukaryotic cells or cultured cell lines in which telomerase activity is not found, such as SaOS2 cells or the like, making the cells or cell lines transiently express USP21 and TERT, and measuring telomerase activity in nuclear extracts of the cells or cell lines (refer to Example 6). The telomerase activity can be measured by a well-known method of measuring telomerase activity such as a TRAP method. NEDD8 conjugation to TERT in cells is performed by an internal NEDD8 conjugation system. Where the telomerase activity in nuclear extracts is increased or generated, after carrying out NEDD8 deconjugation, it can be determined that NEDD8-conjugated TERT underwent NEDD8 deconjugation by USP21. Alternatively, detection of NEDD8 deconjugation by USP21 from NEDD8-conjugated TERT can be carried out also, for example, by using cells or cultured cell lines which show native telomerase activity, such as HEK293T cells or the like, making the cells or cell lines transiently express USP21, and measuring telomerase activity in nuclear extracts of the cells or cell lines (refer to Example 7).

Detection of inhibition of NEDD8 deconjugation by USP21 from NEDD8-conjugated TERT can be carried out, for example, by using eukaryotic cells or cultured cell lines in which expression of TERT or telomerase activity is found, making the cells or cell lines transiently express USP21, and measuring the amount of NEDD8-conjugated TERT and/or the amount of TERT in cell lysates of the cells or cell lines (refer to Example 3). The amount of NEDD8-conjugated TERT and the amount of TERT can be measured by, for example, Western blotting and the like that are well-known in the art. The telomerase activity can be measured by a well-known method of measuring telomerase activity such as a TRAP method. NEDD8 conjugation to TERT in the cells is performed by an internal NEDD8 conjugation system. After carrying out inhibition of NEDD8 deconjugation, the result is compared to the case in which inhibition of NEDD8 deconjugation was not carried out. Where the amount of NEDD8-conjugated TERT in a cell lysate is increased or generated, or where the amount of TERT in a cell lysate is diminished or eliminated, it can be determined that NEDD8 deconjugation by USP21 from NEDD8-conjugated TERT was inhibited. Regarding cells for use, cells that are made to transiently express NEDD8-conjugated TERT can also be used. The expression of NEDD8-conjugated TERT in cells can be carried out using an adequate vector containing a polynucleotide encoding TERT and an adequate vector containing a polynucleotide encoding NEDD8, by transfecting them into cells by conventional genetic engineering techniques.

Detection of inhibition of NEDD8 deconjugation by USP21 from NEDD8-conjugated TERT can be carried out, for example, by using eukaryotic cells or cultured cell lines in which telomerase activity is not found, such as SaOS2 cells or the like, making the cells or cell lines transiently express USP21 and TERT, and measuring telomerase activity in nuclear extracts of the cells or cell lines (refer to Example 6). The telomerase activity can be measured by a well-known method of measuring telomerase activity such as a TRAP method. NEDD8 conjugation to TERT in cells is performed by an internal NEDD8 conjugation system. After carrying out inhibition of NEDD8 deconjugation, the result is compared to the case in which inhibition of NEDD8 deconjugation was not carried out. Where the telomerase activity in nuclear extracts is reduced or eliminated, it can be determined that NEDD8 deconjugation by USP21 from NEDD8-conjugated TERT was inhibited. Alternatively, detection of inhibition of NEDD8 deconjugation by USP21 from NEDD8-conjugated TERT can also be carried out by, for example, using cells or cultured cell lines which show native telomerase activity, such as HEK293T cells or the like, making the cells or cell lines transiently express USP21, and measuring telomerase activity in nuclear extracts of the cells or cell lines (refer to Example 7).

The phrase "binding of USP21 to TERT" refers to the interaction of USP21 with TERT so as to form a complex by a non-covalent bond, such as a hydrogen bond, hydrophobic bond, electrically static interaction or the like. The binding of USP21 to TERT only at a portion of these molecules constitutes "binding" as mentioned herein. For example, an amino acid which is not involved in the binding of USP21 to TERT may be one of the amino acids that constitute USP21 or TERT.

The phrase "binding of USP21 to NEDD8-conjugated TERT" refers to the interaction of USP21 with NEDD8-conjugated TERT so as to form a complex by a non-covalent bond, such as a hydrogen bond, hydrophobic bond, electrically static interaction or the like. The binding of USP21 to NEDD8-conjugated TERT only at a portion of these molecules constitutes "binding" as mentioned herein. For example, an amino acid which is not involved in the binding of USP21 to NEDD8-conjugated TERT may be one of the amino acids that constitute USP21 or NEDD8-conjugated TERT.

The present invention revealed the binding of USP21 to TERT (refer to Example 2). In addition, the present invention demonstrated the NEDD8 deconjugation by USP21 from NEDD8-conjugated TERT (refer to Example 3). These findings show that USP21 binds to TERT, and that TERT then undergoes NEDD8 conjugation by a NEDD8 conjugation system in cells, resulting in generation of NEDD8-conjugated TERT that subsequently undergoes NEDD8 deconjugation by the USP21. Accordingly, the binding of USP21 to NEDD8-conjugated TERT can be, for example, binding of USP21 or a part of USP21 to TERT that constitutes NEDD8-conjugated TERT or a part of TERT that constitutes NEDD8-conjugated TERT.

The inhibition of the binding of USP21 to TERT or the inhibition of the binding of USP21 to NEDD8-conjugated TERT leads to inhibition of NEDD8 deconjugation by USP21 from NEDD8-conjugated TERT. As a result, proteasome-dependent degradation of NEDD8-conjugated TERT is enhanced, which leads to enhanced degradation of TERT and consequent inhibition of telomerase activity.

Detection of the binding of USP21 to NEDD8-conjugated TERT or TERT can be carried out by a method well-known in the art, such as an immuno-precipitation method, a two-hybrid method, a Western blotting, a fluorescence resonance energy transfer method and the like, or by using these methods in combination. NEDD8-conjugated TERT can be obtained, for example, by making cells such as HEK293 T cells transiently express NEDD8 and TERT to generate NEDD8-conjugated TERT by a NEDD8 conjugation system in cells. NEDD8-conjugated TERT contained in the cells can be purified, for example, from cell lysates of the cells by an immuno-precipitation method, an affinity chromatography, or the like, using anti-TERT antibodies and anti-NEDD8 antibodies.

When carrying out the detection of USP21 to NEDD8-conjugated TERT or TERT, it is preferable not to add reducing agents such as dithiothreitol (DTT) or β-mercaptoethanol to the binding reaction mixture so as to inhibit the progression of the NEDD8 deconjugation reaction after binding of USP21 to NEDD8-conjugated TERT. Alternatively, it is preferable to use a USP21 mutant that is an inactive USP21 mutant having binding activity to NEDD8-conjugated TERT. Such a mutant is exemplified by a USP21 mutant in which cysteine at the position of 221 that is an active center for the cysteine protease activity of USP21 is substituted with other amino acid such as alanine or serine.

Detection of inhibition of the binding of USP21 to NEDD8-conjugated TERT or TERT can be carried out by the aforementioned methods of detecting the binding of USP21 to NEDD8-conjugated TERT or TERT. For example, in the case where a signal generated by the binding of USP21 to NEDD8-conjugated TERT or TERT is reduced or eliminated in comparison to that observed before the inhibition of the binding, it can be determined that the binding was inhibited.

TERT, NEDD8, USP21 and genes thereof are not limited to those shown by the aforementioned sequences. Proteins or genes that have one or several mutations in the aforementioned sequences, as long as they have functions that are generally well-known in the art, may also be used. In addition, mutants having one or several introduced mutations for the purpose of facilitating or eliminating those functions can also be used.

Genes respectively encoding TERT, NEDD8 and USP21 can be easily obtained from suitable origins in which expressions of the respective genes are found, for example, human brain, kidney, thymus, and cells derived from breast cancer, by using a cloning method known by itself and the like. Proteins encoded by those genes can be obtained utilizing the respective genes by using known genetic engineering techniques by itself and the like. For example, NEDD8-conjugated TERT can be prepared from cell lysates of cells in which NEDD8-conjugated TERT is expressed, as immuno-precipitates obtained by using anti-TERT antibodies and the like. The expression of NEDD8-conjugated TERT can be carried out by introducing a recombinant vector containing a polynucleotide encoding NEDD8 and a recombinant vector containing a polynucleotide encoding TERT into cells using a well-known method. The NEDD8 and TERT expressed in the cells generate NEDD8-conjugated TERT in the cells via an internal NEDD8 conjugation system in the cells. Cells having a NEDD8 conjugation system are exemplified by HEK293T cells.

Proteasome can be prepared from cells having proteasome. Cells preferably used are, for example, human erythorocytes. The preparation of proteasome can be carried out according to the method of Emmerich et al. (The Journal of Biological Chemistry, 2000, Vol. 275, p. 21140-21148). Alternatively, commercially available proteasome can be used. For example, proteasome can be purchased from AFFINITI Research Products Ltd., UK.

The inhibition of NEDD8 deconjugation by USP21 from NEDD8-conjugated TERT can be carried out, for example, by utilizing a compound that inhibits the NEDD8 deconjugation by USP21 from NEDD8-conjugated TERT. The inhibition of the binding of USP21 to NEDD8-conjugated TERT can be carried out, for example, by utilizing a compound that inhibits the binding of USP21 to NEDD8-conjugated TERT. The inhibition of the binding of USP21 to TERT can be carried out, for example, by utilizing a compound that inhibits the binding of USP21 to TERT. As used herein, a compound having such an inhibitory effect (such as polypeptides having a competitive inhibitory effect, antibody and compound having a lower molecular weight and the like, which are listed in the example below) may be referred to as an inhibitor.

A compound that inhibits the NEDD8 deconjugation by USP21 from NEDD8-conjugated TERT can be, for example, a compound capable of reducing cysteine protease activity of USP21. Examples of such a compound include cystein protease inhibitors. The cystein protease inhibitors capable of specifically inhibiting the cysteine protease activity of USP21 are preferably used. The phrase "specifically inhibiting the cysteine protease activity of USP21" refers to inhibiting the cysteine protease activity of USP21 strongly, but not inhibiting or weakly inhibiting the enzyme activity of the other enzymes.

A compound that inhibits the NEDD8 deconjugation by USP21 from NEDD8-conjugated TERT can be, for example, a compound capable of inhibiting the binding of USP21 to NEDD8-conjugated TERT. A compound capable of inhibiting the binding of USP21 to NEDD8-conjugated TERT can be preferably a compound capable of specifically inhibiting the binding, and more preferably a low molecular weight compound capable of specifically inhibiting the binding. The phrase "specifically inhibiting the binding of USP21 to NEDD8-conjugated TERT" refers to strongly inhibiting the binding of USP21 to NEDD8-conjugated TERT, but not inhibiting or weakly inhibiting the binding between other proteins.

Further, a compound that inhibits the NEDD8 deconjugation by USP21 from NEDD8-conjugated TERT can be, for example, a compound capable of inhibiting the binding of USP21 to TERT. A compound capable of inhibiting the binding of USP21 to TERT can be preferably a compound capable of specifically inhibiting the binding, and more preferably a low molecular weight compound capable of specifically inhibiting the binding of USP21 to TERT. The phrase "specifically inhibiting the binding of USP21 to TERT" refers to strongly inhibiting the binding, but not inhibiting or weakly inhibiting the binding between other proteins.

A compound that inhibits the binding of USP21 to NEDD8-conjugated TERT can be exemplified by inactive USP21 mutants. Preferable examples of the inactive mutants include an inactive mutant having binding activity to NEDD8-conjugated TERT. Such an inactive mutant binds to NEDD8-conjugated TERT in a competitive manner with wild type USP21, and is thereby capable of inhibiting the action of USP21 on NEDD8-conjugated TERT. Such an inactive mutant can be identified by carrying out a method of identifying a compound that inhibits the binding of USP21 to NEDD8-conjugated TERT described later.

A compound that inhibits the binding of USP21 to TERT can be exemplified by inactive USP21 mutants. Preferable examples of the inactive mutants include an inactive mutant having binding activity to TERT. Such an inactive mutant binds to TERT in a competitive manner with wild type USP21, and is thereby capable of inhibiting the action of USP21 on TERT. Such an inactive mutant can be identified by carrying out a method of identifying a compound that inhibits the binding of USP21 to TERT described later.

The term "inactive USP21 mutants" refers to USP21 having introduced mutations of amino acids, such as deletion, substitution, addition, or insertion, which shows reduced or eliminated cysteine protease activity in comparison to wild type USP21. The inactive USP21 mutants can be those existing in nature, or can be those having artificially introduced mutations. Techniques for introducing such a mutation are well-known in the art. For example, the Ulmer's technique (K. M. Ulmer, Science, 1983, Vol. 219, p.666-671) can be employed as such techniques. The position of mutation in the amino acid sequence of USP21 can be, for example, a position necessary for cysteine protease activity of USP21. Specifically, the position can be a cysteine residue at the position of 221. The cysteine residue, an active center of the cystein protease, is an essential amino acid residue for cysteine protease activity of USP21 (Non-patent Reference 9).

The binding of an inactive USP21 mutant (hereinafter, may be referred to as an inactive USP21) to NEDD8-conjugated TERT can be detected by a method similar to the aforementioned methods of detecting the binding of USP21 to NEDD8-conjugated TERT.

A compound that inhibits the binding of USP21 to NEDD8-conjugated TERT can be exemplified by a polypeptide consisting of the amino acid sequence of the region where USP21 binds to NEDD8-conjugated TERT. A compound that inhibits the binding of USP21 to TERT can be exemplified by a polypeptide consisting of the amino acid sequence of the region where USP21 binds to TERT. Such polypeptides can inhibit the binding between proteins in a competitive manner. Such polypeptides can be obtained by constructing peptides based on the amino acid sequence of USP21 or TERT, synthesizing by well-known peptide synthesis methods, and selecting peptides that inhibit the binding of USP21 to NEDD8-conjugated TERT or the binding of USP21 to TERT. A polypeptide prepared by introducing mutations such as deletion, substitution, addition or insertion of one or several amino acids into the thus specified peptide is also included in the scope of the present invention. Such a polypeptide having introduced mutations is preferably a polypeptide that inhibits the binding of USP21 to NEDD8-conjugated TERT or the binding of USP21 to TERT. The polypeptide having mutations can be those existing in nature, or can be those having artificially introduced mutations. Techniques for introducing such a mutation are well-known in the art. For example, the Ulmer's technique (K. M. Ulmer, Science, 1983, Vol. 219, p.666-671) can be employed as one such technique. When introducing a mutation, in view of avoiding a change in the fundamental properties of the polypeptide (such as physical properties, function, immunological activity, or the like), mutual substitution among homologous amino acids (polar amino acids, non-polar amino acids, hydrophobic amino acids, hydrophilic amino acids, positively-charged amino acids, negatively-charged amino acids and aromatic amino acids or the like) is readily conceivable. Furthermore, these usable polypeptides can be modified to the extent that no significant functional change is involved, for example, modification by amidation of its constituent amino group or carboxyl group and the like.

The aforementioned peptides can be produced by common methods that are known in peptide chemistry. A method described in the References ("PEPUTIDO GOUSEI" (Japan), Maruzen Co., Ltd., 1975; "Peptide Synthesis" (USA), Interscience, 1996), for example, may be used. However, the methods are not limited thereto and any known methods can be broadly utilized. Specifically, a peptide synthesis method using an ordinary liquid phase method and solid phase method, such as, for example, the Fmoc method, can be exemplified. Moreover, an amino acid synthesizer that is commercially available can be used for producing the polypeptide. Alternatively, genetic engineering techniques can also be used for producing the polypeptide. For example, a gene encoding a polypeptide of interest can be used to prepare a recombinant expression vector that can express the gene in a host cell, followed by transfection of the recombinant expression vector into a suitable host cell such as E. coli to obtain a transformant. Culturing the transformant and collecting the polypeptide of interest from the obtained culture product achieves the production of the polypeptide.

The inhibition of the binding of USP21 to NEDD8-conjugated TERT can also be carried out by using antibodies that recognize USP21 or NEDD8-conjugated TERT and inhibit the binding of USP21 to NEDD8-conjugated TERT. Such antibodies can be produced by known methods for preparing antibodies, using antigens such as USP21 or NEDD8-conjugated TERT itself, or a fragment of these proteins, preferably a polypeptide consisting of the amino acid sequence of the region where USP21 binds to NEDD8-conjugated TERT.

The inhibition of the binding of USP21 to TERT can also be carried out using antibodies that recognize USP21 or TERT and inhibit the binding of USP21 to TERT. Such antibodies can be produced by known methods for preparing antibodies, using antigens such as USP21 or TERT itself, or a fragment of these proteins, preferably a polypeptide consisting of the amino acid sequence of the region where USP21 binds to TERT.

The inhibition of the binding of USP21 to NEDD8-conjugated TERT can also be carried out using a compound that inhibits the expression of USP21. Examples of compounds that inhibit the expression of USP21 include antisense oligonucleotides of USP gene. Further, siRNA (small interfering RNA) capable of reducing or eliminating the expression of USP21 through RNA interference can also be included in the examples of compounds that inhibit the binding of USP21 to NEDD8-conjugated TERT.

The inhibition of the binding of USP21 to TERT can also be carried out using a compound that inhibits the expression of USP21. Examples of a compound that inhibits the expression of USP21 include antisense oligonucleotides of the USP gene. Further, siRNA (small interfering RNA) capable of reducing or eliminating the expression of USP21 through RNA interference can also be included in the examples of compounds that inhibit the binding of USP21 to TERT.

A still further aspect of the present invention relates to a method of inhibiting telomerase activity which comprises utilizing the method of enhancing degradation of TERT according to the present invention.

The phrase "inhibiting telomerase activity" refers to reducing or eliminating telomerase activity by degradation of TERT, a component of telomerase, in comparison to telomerase activity before degradation of TERT. Telomerase is a ribonucleic protein comprising telomerase RNA that is a template RNA molecule and TERT that is a catalytic subunit. The present inventors found out that there probably exists a proteasome-dependent regulatory system for TERT degradation via NEDD8 conjugation to TERT and NEDD8 deconjugation by USP21 from NEDD8-conjugated TERT, and that the regulatory system for TERT degradation probably regulates nuclear telomerase activity in cells. On the other hand, it has been reported that SaOS2 cells (human osteosarcoma cells) expressing TR gene only but not TERT gene do not have telomerase activity. This indicates that telomerase activity is regulated by the amount of TERT. Accordingly, the method of enhancing degradation of TERT according to the present invention can be used for reducing the amount of TERT in cells, thereby allowing inhibition of telomerase activity.

A further aspect of the present invention relates to a method of identifying a compound that inhibits binding of USP21 to NEDD8-conjugated TERT. The identification method of the compound can be carried out by employing any known pharmaceutical screening system. For example, the compound that inhibits binding of USP21 to NEDD8-conjugated TERT can be identified by contacting USP21 and/or NEDD8-conjugated TERT with a compound to be tested (hereinafter, may be referred to as a test compound), under selected conditions allowing the interaction of USP21 and/or NEDD8-conjugated TERT with the test compound, employing a system that uses a signal and/or a marker capable of detecting the binding of USP21 to NEDD8-conjugated TERT, and then detecting the presence, the absence or the change of the signal and/or the marker.

A still further aspect of the present invention relates to a method of identifying a compound that inhibits binding of USP21 to TERT. The identification method of the compound can be carried out by employing any known pharmaceutical screening system. For example, the compound that inhibits binding of USP21 to TERT can be identified by contacting USP21 and/or TERT with a compound to be tested, under selected conditions allowing the interaction of USP21 and/or TERT with the test compound, employing a system that uses a signal and/or a marker capable of detecting the binding of USP21 to TERT, and then detecting the presence, the absence or the change of the signal and/or the marker.

In the method of identifying a compound that inhibits binding of USP21 to NEDD8-conjugated TERT, the test compound may be previously contacted with USP21 and/or NEDD8-conjugated TERT, and then the binding reaction of USP21 to NEDD8-conjugated TERT may be conducted. Alternatively, the test compound may be allowed to coexist in the binding reaction of these proteins. The conditions that allow the interaction of a test compound with USP21 and/or NEDD8-conjugated TERT may be a condition *in vitro or in vivo.* For example, eukaryotic cells or cultured cell lines in which expression of TERT or telomerase activity is found may be used. Alternatively, cells in which USP21 is co-expressed with NEDD8-conjugated TERT may also be used. The expression in cells can be carried out using an adequate vector containing a polynucleotide encoding USP21, an adequate vector containing a polynucleotide encoding TERT, and an adequate vector containing a polynucleotide encoding NEDD8, by transfecting these vectors into cells by conventional genetic engineering techniques.

Where the signal that is generated from the binding of USP21 to NEDD8-conjugated TERT or the marker of the binding of USP21 to NEDD8-conjugated TERT changes, for example, reduces or disappears when contacting a test compound with USP21 and/or NEDD8-conjugated TERT in the method of identifying a compound that inhibits binding of USP21 to NEDD8-conjugated TERT, it can be determined that the test compound inhibits the binding of USP21 to NEDD8-conjugated TERT. The binding of USP21 to NEDD8-conjugated TERT can be determined easily by detecting the presence or absence of the binding of these proteins and/or measuring changes in binding amount. Detection of the binding of these proteins can be carried out using well-known methods for detecting proteins, such as Western blotting and the like.

In the method of identifying a compound that inhibits binding of USP21 to TERT, the test compound may be previously contacted with USP21 and/or TERT, and then the binding reaction of USP21 to TERT may be conducted. Alternatively, the test compound may be allowed to coexist in the binding reaction of these proteins. The conditions that allow the interaction of a test compound with USP21 and/or TERT may be a condition *in vitro or in vivo.* For example, eukaryotic cells or cultured cell lines in which expression of TERT or telomerase activity is found may be used. Alternatively, cells in which USP21 is co-expressed with TERT may also be used. The expression in cells can be carried out using an adequate vector containing a polynucleotide encoding USP21 and an adequate vector containing a polynucleotide encoding TERT, by transfecting these vectors into cells by conventional genetic engineering techniques.

Where the signal that is generated from the binding of USP21 to TERT or the marker of the binding of USP21 to TERT changes, for example, reduces or disappears when contacting a test compound with USP21 and/or TERT in the method of identifying a compound that inhibits binding of USP21 to TERT, it can be determined that the test compound inhibits the binding of USP21 to TERT. The binding of USP21 to TERT can be determined easily by detecting the presence or absence of the binding of these proteins and/or measuring changes in binding amount. Detection of the binding of these proteins can be carried out using well-known methods for detecting proteins, such as Western blotting and the like.

The method of identifying a compound that inhibits binding of USP21 to NEDD8-conjugated TERT can be carried out, for example, using an experimental system which brings GST fused USP21 into reaction with NEDD8-conjugated TERT followed by detecting the binding of these proteins by a pull down method using glutathione resin. Detection of the binding of these proteins can be carried out, for example, using Western blotting and the like.

When carrying out such an experimental system, it is preferable not to add reducing agents that are necessary for cysteine protease activity of USP21, such as dithiothreitol (DTT) or β-mercaptoethanol, to the experimental system, in order to inhibit or terminate the USP21-induced release of NEDD8 from NEDD8-conjugated TERT after the binding of USP21 to NEDD8-conjugated TERT. Alternatively, it is preferable to use a USP21 mutant that is an inactive USP21 mutant having binding activity to NEDD8-conjugated TERT. Such a mutant is exemplified by a USP21 mutant in which cysteine at the position of 221 that is an active center for the cysteine protease activity of USP21 is substituted with other amino acid such as alanine or serine.

Where the amount of binding between these proteins detected in the presence of a test compound is reduced or eliminated in comparison to the amount of binding detected in the absence of the test compound in such an experimental system, it can be determined that the test compound inhibits the binding.

The present invention revealed the binding of USP21 to TERT (refer to Example 2). In addition, the present invention demonstrated NEDD8 deconjugation by USP21 from NEDD8-conjugated TERT (refer to Example 3). From these findings, it can be concluded that USP21 binds to TERT, and that TERT then undergoes NEDD8 conjugation by a NEDD8 conjugation system in cells resulting in generation of NEDD8-conjugated TERT that subsequently undergoes NEDD8 deconjugation by the USP21. Therefore, it can be concluded that inhibition of the binding of USP21 to TERT allows inhibition of the binding of USP21 to NEDD8-conjugated TERT. Accordingly, the present identification methods can also be carried out by using TERT instead of NEDD8-conjugated TERT.

The method of identifying a compound that inhibits the binding of USP21 to NEDD8-conjugated TERT can be carried out, for example, using a method of detecting the binding of USP21 to TERT. Specifically, the method can be carried out by employing an experimental system which uses cells that are prepared by expressing both USP21 labeled with a labeling substance and TERT labeled with another labeling substance, and detects intracellular binding reaction (refer to Example 2). Detection of the binding of USP21 to TERT in such an experimental system can be carried out by well-known methods such as an immunoprecipitation method, Western blotting or the like. Where the binding is not detected in the presence of a test compound or the amount of binding proteins is reduced in comparison to a result obtained in the absence of the test compound in such an experimental system, it can be determined that the test compound inhibits the binding of USP21 to TERT and the binding of USP21 to NEDD8-conjugated TERT.

The method of identifying a compound that inhibits the binding of USP21 to NEDD8-conjugated TERT can also be carried out, using an experimental system which brings GST fused USP21 into reaction with TERT followed by detecting the binding of these proteins by a pull down method using glutathione resin (refer to Example 2). Detection of the binding of these proteins can be carried out, for example, using Western blotting and the like. Detection of the binding of GST fused USP21 to TERT in such an experimental system can be carried out by (i) using TERT previously labeled with a labeling substance, (ii) bringing GST fused USP21 into reaction with the TERT, (iii) mixing the reaction product with glutathione sepharose, (iv) fractionating a complex of GST fused USP21 and TERT, and then (v) detecting the labeling substances contained in the complex. Any labeling substances can be used as long as they are labeling substances conventionally used for detection of proteins. For example, radio isotopes such as 35S and the like are preferably used. Where the labeling substances is not detected in the presence of a test compound or the detected amount of the labeling substances is reduced in comparison to a result obtained in the absence of a test compound in such an experimental system, it can be determined that the test compound inhibits the binding of USP21 to TERT and the binding of USP21 to NEDD8-conjugated TERT.

The method of identifying a compound that inhibits binding of USP21 to NEDD8-conjugated TERT can also be carried out, using the known two-hybrid method for the experimental system. For example, the method can be achieved by transfecting yeasts, eukaryotic cells, or the like, with a plasmid for expressing USP21 as a fusion protein with a DNA binding protein, a plasmid for expressing TERT as a fusion protein with a transcription activating protein, and a plasmid containing a reporter gene connected with a suitable promoter gene, and then comparing an expression amount of the reporter gene in the presence of a test compound to an expression amount of the reporter gene in the absence of the test compound. Where the expression amount of the reporter gene in the presence of a test compound is reduced in comparison to the expression amount of the reporter gene in the absence of the test compound, it can be determined that the test compound has an effect of inhibiting the binding of USP21 to TERT and the binding of USP21 to NEDD8-conjugated TERT. Examples of a reporter gene include lacZ.

A further aspect of the present invention relates to a method of identifying a compound that inhibits NEDD8 deconjugation by USP21 from NEDD8-conjugated TERT. The identification method of the compound can be carried out by employing any known pharmaceutical screening system. For example, the compound that inhibits NEDD8 deconjugation by USP21 from NEDD8-conjugated TERT can be identified by contacting a compound to be tested (hereinafter, may be referred to as a test compound) with USP21 and/or NEDD8-conjugated TERT, under selected conditions allowing the interaction of the test compound with USP21 and/or NEDD8-conjugated TERT, employing a system that uses a signal and/or a marker capable of detecting the NEDD8 deconjugation by USP21 from NEDD8-conjugated TERT, and then detecting the presence, the absence or the change of the signal and/or the marker.

In the method of identifying a compound that inhibits NEDD8 deconjugation by USP21 from NEDD8-conjugated TERT, the test compound may be previously contacted with USP21 and NEDD8-conjugated TERT, and then the NEDD8 deconjugation reaction by USP21 from NEDD8-conjugated TERT may be conducted. Alternatively, the test compound may be allowed to coexist in the reaction. The conditions that allow the interaction of a test compound with USP21 and NEDD8-conjugated TERT may be *in vitro or in vivo* conditions. For example, eukaryotic cells or cultured cell lines in which expression of TERT or telomerase activity is found may be used. Alternatively, cells in which USP21 is co-expressed with NEDD8-conjugated TERT may also be used. The expression in cells can be carried out using an adequate vector containing a polynucleotide encoding USP21, an adequate vector containing a polynucleotide encoding TERT, and an adequate vector containing a polynucleotide encoding NEDD8, and by transfecting these vectors into cells by conventional genetic engineering techniques.

Where the signal that is generated from NEDD8 deconjugation by USP21 from NEDD8-conjugated TERT or the marker of the NEDD8 deconjugation by USP21 from NEDD8-conjugated TERT changes, for example, reduces or disappears when contacting a test compound with USP21 and/or NEDD8-conjugated TERT in the method of identifying a compound that inhibits NEDD8 deconjugation by USP21 from NEDD8-conjugated TERT, it can be determined that the test compound inhibits NEDD8 deconjugation by USP21 from NEDD8-conjugated TERT.

The present invention provided a presumption that USP21 binds to TERT, and that the TERT then undergoes NEDD8 conjugation by a NEDD8 conjugation system in cells resulting in generation of NEDD8-conjugated TERT that subsequently undergoes NEDD8 deconjugation by USP21 resulting in a consequent reduction in the amount of NEDD8-conjugated TERT. Therefore, the inhibition of NEDD8 deconjugation by USP21 from NEDD8-conjugated TERT can be detected by the amount of NEDD8-conjugated TERT and the amount of TERT. Accordingly, the amount of NEDD8-conjugated TERT and the amount of TERT can be used as an indicator in the method of identifying a compound that inhibits NEDD8 deconjugation by USP21 from NEDD8-conjugated TERT. In the case of carrying out the identification method using the amount of NEDD8-conjugated TERT as an indicator, where the amount of NEDD8-conjugated TERT is increased or generated by a test compound in comparison to that observed before using the test compound, it can be determined that the test compound inhibits NEDD8 deconjugation by USP21 from NEDD8-conjugated TERT. In the case of carrying out the identification method using the amount of TERT as an indicator, where the amount of TERT is eliminated or decreased by a test compound in comparison to that observed before using the test compound, it can be determined that the test compound inhibits NEDD8 deconjugation by USP21 from NEDD8-conjugated TERT.

The method of identifying a compound that inhibits NEDD8 deconjugation by USP21 from NEDD8-conjugated TERT can be carried out, for example, using an experimental system which detects NEDD8-conjugated TERT and TERT in cell lysates prepared from cells in which TERT was transiently co-expressed with NEDD8, by Western blotting (refer to Example 3). Detection of NEDD8-conjugated TERT and TERT in such an experimental system can be carried out by detecting a labeling substance with which NEDD8 and/or TERT were previously labeled. A labeling substance is preferably exemplified by HA-tag, FLAG-tag, and the like. Where the amount of NEDD8-conjugated TERT detected in the presence of a test compound is generated or increased in comparison to the amount of NEDD8-conjugated TERT detected in the absence of a test compound, it can be determined that the test compound inhibits NEDD8 deconjugation by USP21 from NEDD8-conjugated TERT. Alternatively, where the amount of TERT detected in the presence of a test compound is eliminated or decreased in comparison to the amount of TERT detected in the absence of a test compound, it can be determined that the test compound inhibits NEDD8 deconjugation by USP21 from NEDD8-conjugated TERT. In such cases, it is preferable that the total amount of the two proteins, NEDD8-conjugated TERT and TERT, detected in the presence of a test compound is substantially equal to that of these proteins detected in the absence of a test compound.

The present invention provided a presumption that USP21 binds to TERT, and that the TERT then undergoes NEDD8 conjugation by a NEDD8 conjugation system in cells resulting in generation of NEDD8-conjugated TERT that subsequently undergoes NEDD8 deconjugation by the USP21 resulting in a consequent reduction in the amount of NEDD8-conjugated TERT and an increase in the amount of TERT. The present invention also provided a presumption that TERT undergoes NEDD8-conjugation and is thereby degraded by proteasome. Since TERT is a catalytic subunit of telomerase, it can be considered that an increase in the amount of TERT leads to enhancement of nuclear telomerase activity. Therefore, NEDD8 deconjugation from NEDD8-conjugated TERT and the inhibition of the NEDD8 deconjugation can be detected by monitoring telomerase activity. Accordingly, the method of identifying a compound that inhibits NEDD8 deconjugation by USP21 from NEDD8-conjugated TERT can be carried out by using telomerase activity as an indicator. In the case of carrying out the identification method by using telomerase activity as an indicator, where the telomerase activity is eliminated or decreased by a test compound in comparison to that observed before using the test compound, it can be determined that the test compound inhibits NEDD8 deconjugation by USP21 from NEDD8-conjugated TERT.

The method of identifying a compound that inhibits NEDD8 deconjugation by USP21 from NEDD8-conjugated TERT can be carried out, for example, by using an experimental system which measures telomerase activity in nuclear extracts prepared from cells in which USP21 was transiently co-expressed with TERT, and well known methods for measuring telomerase activity (refer to Example 6). The methods for measuring telomerase activity are preferably exemplified by the TRAP method. Regarding cells for use, preferably, animal cells that do not show native telomerase activity can be used. More preferably, SaOS 2 cells (human osteosarcoma cell line) can be used. Where the telomerase activity detected in the presence of a test compound is eliminated or reduced in comparison to the telomerase activity detected in the absence of the test compound in such an experimental system, it can be determined that the test compound inhibits NEDD8 deconjugation by USP21 from NEDD8-conjugated TERT.

The method of identifying a compound that inhibits NEDD8 deconjugation by USP21 from NEDD8-conjugated TERT can be carried out, for example, using an experimental system which measures telomerase activity in nuclear extracts prepared from cells in which USP21 was transiently expressed and well-known methods for measuring telomerase activity (refer to Example 7). The methods for measuring telomerase activity are preferably exemplified by the TRAP method. Regarding cells for use, preferably, animal cells that show native telomerase activity can be used. More preferably, HEK293T cells can be used. Where the telomerase activity detected in the presence of a test compound is eliminated or reduced in comparison to the telomerase activity detected in the absence of the test compound in such an experimental system, it can be determined that the test compound inhibits NEDD8 deconjugation by USP21 from NEDD8-conjugated TERT.

TERT, NEDD8 and USP21 may be prepared from cells in which they are expressed by means of genetic engineering techniques, or from any suitable biological samples. These proteins also may be products of a cell-free synthesis system, or chemical synthesis products. These proteins can be subsequently further purified for use. Alternatively, cells in which TERT, NEDD8 and USP21 are expressed by means of genetic engineering techniques may be used. TERT, NEDD8 and USP21 can be modified, to the extent that no significant characteristic and functional change is involved, by adding other proteins or polypeptides, for example, GST, β-galactosidase, Fc fragments of immunogloblin such as IgG, or tag-peptides such as His-tag, Myc-tag, HA-tag, FLAG-tag or Xpress-tag, to the N-terminal or C-terminal, directly or indirectly via a linker peptide or the like, by means of gene manipulation techniques or the like. The features or functions of TERT, NEDD8 and USP21 are, for example, the binding of TERT to NEDD8, the binding of TERT to USP21, the binding of NEDD8-conjugated TERT to NUB1, the binding of NEDD8-conjugated TERT to S5a, the binding of NEDD8-conjugated TERT to USP21, the cysteine protease activity of USP21, NEDD8 deconjugation by USP21 from NEDD8-conjugated TERT.

Examples of test compounds include a compound derived from a chemical library or natural products, as well as a compound obtained by drug design based on the primary structure or tertiary structure of TERT, USP21 and NEDD8. Alternatively, compounds obtained by drug design based on the structure of a polypeptide that comprises an amino acid sequence of a binding region of TERT to USP21 are also suitable for test compounds.

A still further aspect of the present invention relates to an agent for inhibiting telomerase activity which contains a compound that enhances a degradation of TERT. The agent for inhibiting telomerase activity can be an agent for inhibiting telomerase activity which contains a compound that inhibits NEDD8 deconjugation by USP21 from NEDD8-conjugated TERT. The agent for inhibiting telomerase activity can be an agent for inhibiting telomerase activity which contains a compound that inhibits the binding of USP21 to NEDD8-conjugated TERT. The agent for inhibiting telomerase activity can be an agent for inhibiting telomerase activity which contains a compound that inhibits the binding of USP21 to TERT.

A further aspect of the present invention relates to pharmaceutical compositions which contain the aforementioned compound and the aforementioned agent for inhibiting telomerase activity. The aforementioned compounds and the aforementioned agents for inhibiting telomerase activity are selected based on the balance between usefulness and toxicity, and can be utilized for preparing pharmaceutical compositions. In preparing the pharmaceutical compositions, these compounds and inhibitors may be used alone or in combination. The aforementioned agents for inhibiting telomerase activity and pharmaceutical compositions can be utilized as an agent for preventing and/or treating diseases attributable to telomerase action or enhanced telomerase activity. Further, the aforementioned agents for inhibiting telomerase activity and pharmaceutical compositions can be utilized for carrying out a method of preventing and/or treating such diseases. The method of preventing and/or treating such diseases can also be carried out by employing the aforementioned method of inhibiting telomerase activity. Examples of the diseases attributable to the enhanced telomerase activity include cancer diseases. In other words, the aforementioned agents for inhibiting telomerase activity and the aforementioned pharmaceutical compositions can be utilized as anti-cancer agents. In addition, the aforementioned agents for inhibiting telomerase activity, the aforementioned pharmaceutical compositions and the aforementioned methods of inhibiting telomerase activity can be utilized for carrying out a method of preventing and/or treating diseases attributable to the enhanced telomerase activity, such as cancer diseases.

USP21 and NEDD8 are ubiquitous proteins which are expressed in various tissues derived from a living human body. The present invention provides examples utilizing HeLa cells originating in human uterus cancer tissues and provides a presumption that there exists a proteasome-dependent degradation system for NEDD8-conjugated TERT and of TERT in the cells. Further, the expression of USP21 in the cells was detected by using RT-PCR (Example 8). Therefore, the present inventors believe that a proteasome-dependent regulatory system for TERT degradation via NEDD8 conjugation to TERT and NEDD8 deconjugation by USP21 from NEDD8-conjugated TERT exists also in cancer cells, and that the regulatory system for TERT degradation regulates telomerase activity in cancer cells.

Telomerase activity has been found in most tumors regardless of the kinds of tumors. Thus, it is believed that the pharmaceutical compositions according to the present invention are effective in inhibiting proliferation of a variety of cancer cells. Therefore, when using the pharmaceutical compositions for cancer diseases, the application thereof are not particularly limited by the kinds of tumors, and the pharmaceutical compositions can be used for both solid tumors and non-solid tumors. The kinds of solid tumors or non-solid tumors are also not particularly limited, and the pharmaceutical composition can be used for all kinds of tumors having telomerase activity. Examples of cancer diseases include solid tumors such as stomach cancer, esophageal cancer, colon cancer, small intestinal cancer, duodenal carcinoma, lung cancer, liver cancer, gallbladder cancer, pancreatic cancer, renal cancer, bladder cancer, oral cancer, osteocarcinoma, skin cancer, breast cancer, uterine cancer, prostate cancer, brain tumor, neuroblastoma and the like, or non-solid tumors such as leukemia, malignant lymphoma, and the like. However, application of the pharmaceutical compositions is not limited to these diseases.

The agent for preventing and/or treating diseases according to the present invention can be prepared as pharmaceutical compositions containing an effective amount of at least one member selected from the aforementioned compounds and the aforementioned inhibitors, as an effective ingredient. In general, it is preferable to prepare pharmaceutical compositions containing one or more kinds of pharmaceutical carriers in addition to the effective ingredient.

An amount of the effective ingredient contained in the pharmaceutical composition according to the present invention can be suitably selected from a wide range. In general, a suitable amount may fall within a range of approximately 0.00001 to 70 wt%, preferably approximately 0.0001 to 5 wt%.

The pharmaceutical carrier may be a filler, an extender, a binder, a wetting agent, a disintegrator, a lubricant, a diluent, an excipient and the like, which can be generally used depending on the use and rates of administration of the pharmaceutical composition. These carriers can be suitably selected and used depending on how the pharmaceutical composition is used.

Specifically, the pharmaceutical carrier may be, for example, water, a pharmaceutically acceptable organic solvent, collagen, polyvinyl alcohol, polyvinylpyrrolidone, carboxyvinyl polymer, sodium alginate, soluble dextran, sodium carboxymethyl starch, pectin, xanthan gum, acacia gum, casein, gelatin, agar, glycerin, propylene glycol, polyethylene glycol, vaseline, paraffin, stearyl alcohol, stearic acid, human serum albumin, mannitol, sorbitol and lactose. One or a combination of two or more kinds of these carriers may be suitably selected, and used depending on the use of a pharmaceutical composition of the present invention.

As desired, various ingredients used in conventional protein preparations can be suitably used herein, such as, a stabilizer, a bacteriocide, a buffer agent, an isotonizing agent, a chelating agent, a pH adjuster, or a surfactant.

As a stabilizer, the following may be used: human serum albumin, common L-amino acids, sugars, and cellulose derivatives. These can be used independently or in combination with a surfactant, and the like. Use of these in such a combination may give increased stability to an effective ingredient. An L-amino acid is not particularly limited, and may be any one of glycine, cysteine, glutamic acid, and the like. A sugar is not particularly limited, and may be any one of the monosaccharides such as glucose, mannose, galactose and fructose, sugar alcohols such as mannitol, inositol and xylitol, disaccharides such as sucrose, maltose and lactose, polysaccharides such as dextran, hydroxypropylstarch, chondroitin sulfate and hyaluronic acid, derivatives thereof, and the like. A cellulose derivative is not particularly limited, and may be any one of methylcellulose, ethylcellulose, hydroxyethylcellulose, hydroxypropylcellulose, hydroxypropylmethylcellulose, sodium carboxymethylcellulose, and the like.

A surfactant is not particularly limited, and can be both an ionic surfactant and a non-ionic surfactant. As a surfactant, the following may be used: polyoxyethyleneglycol sorbitan alkyl ester base; polyoxyethylene alkyl ether base; sorbitan monoacyl ester base; or a fatty acid glyceride base.

As a buffer agent, the following may be used: boric acid; phosphoric acid; acetic acid; citric acid; ε-aminocaproic acid; glutamic acid; and/or a salt thereof, for example, an alkali metal salt or an alkaline earth metal salt, such as a sodium salt, a potassium salt, a calcium salt and a magnesium salt.

As an isotonizing agent, the following may be used: sodium chloride; potassium chloride; sugars; or glycerin.

As a chelating agent, sodium edentate and citric acid may be used.

The medicaments and the pharmaceutical compositions according to the present invention can be used as solution preparations. Alternatively, they can be freeze-dried, so as to be preservable. They can be used by dissolving them in water, a buffered solution containing saline, and the like, and then adjusting them to a suitable concentration at the time of use.

Suitable dosage ranges of the medicaments and the pharmaceutical compositions are not particularly limited, and can be determined in accordance with the following: effectiveness of the ingredients contained therein; the administration form; the route of administration; the type of disease; the characteristics of the subject (e.g., body weight, age, symptomatic conditions, and whether a subject is taking other pharmaceutical agents); and the judgment of a physician in charge. In general, a suitable dosage may fall, for example, within a range of about 0.01 1µg to 100 mg, per 1 kg of the body weight of the subject, and preferably within a range of about 0.1 µg to 1 mg, per 1 kg of body weight. However, the dosage may be altered using conventional experiments for optimization of a dosage that are well-known in the art. The aforementioned dosage can be divided for administration once to several times a day. Alternatively, periodic administration once every few days or few weeks can be employed.

When administering the pharmaceutical compositions according to the present invention, the pharmaceutical composition may be used alone, or may be used together with other compounds or medicaments necessary for preventing and/or treating the target disease. For example, effective ingredients contained in the other telomerase activity inhibitors or anti-tumor drugs may be used.

In terms of a route of administration, it may be either systemic administration or local administration. The route of administration that is appropriate for a particular disease, symptomatic condition, or other factors, should be considered. For example, parenteral administration including normal intravenous injection, intra-arterial administration, subcutaneous administration, intracutaneous administration, and intramuscular administration can be employed. Oral administration can be also employed. Further, transmucosal administration or dermal administration can be employed. In the case of use for cancer diseases, it may be preferable to employ a direct administration into the tumor by injection, and the like.

In terms of an administration form, various forms can be selected in accordance with a treatment purpose. For example, a solid formulation may be employed such as a tablet, pill, powder, powdered drug, fine granule, granule, or a capsule. Alternatively, a liquid formulation can be employed such as an aqueous formulation, ethanol formulation, suspension, fat emulsion, liposome formulation, clathrate such as cyclodextrin, syrup, or an elixir. These can be further classified, according to the administration route, into an oral formulation, parenteral formulation (drip injection formulation or injection formulation), nasal formulation, inhalant formulation, transvaginal formulation, suppositorial formulation, sublingual agents, eye drop formulation, ear drop formulation, ointment formulation, cream formulation, transdermal absorption formulation, transmucosal absorption formulation, and the like, which can be respectively blended, formed and prepared according to conventional methods.

A still further aspect of the present invention relates to a reagent kit. The reagent kit can be a reagent kit including the following: at least one member selected from USP21, a polynucleotide encoding USP21, a recombinant vector containing the polynucleotide and a transformant containing the recombinant vector; at least one member selected from TERT, a polynucleotide encoding TERT, a recombinant vector containing the polynucleotide and a transformant containing the recombinant vector; and at least one member selected from NEDD8, a polynucleotide encoding NEDD8, a recombinant vector containing the polynucleotide and a transformant containing the recombinant vector. Preferably, the reagent kit includes at least one member selected from an enzyme necessary for NEDD8 conjugation to TERT, a gene encoding the enzyme, a vector containing the gene, and a transformant containing the vector. Further, the reagent kit can be a reagent kit including the following: at least one member selected from TERT, a polynucleotide encoding TERT, a recombinant vector containing the polynucleotide and a transformant containing the recombinant vector; and at least one member selected from NEDD8, a polynucleotide encoding NEDD8, a recombinant vector containing the polynucleotide and a transformant containing the recombinant vector; and proteasome. The reagent kit of the present invention can be used for the method of identifying a compound according to the present invention.

USP21, TERT and NEDD8 can be obtained by the aforementioned production methods. In addition, polynucleotides, recombinant vectors and transformants can be prepared by the aforementioned genetic engineering techniques. Proteasome can be prepared by the aforementioned production methods, or is commercially available for use. The reagent kit of the present invention may include any materials necessary for carrying out the aforementioned identification methods, such as signals and/or markers, buffer solutions, salts, and the like. The reagent kit may further include materials such as stabilizers and/or antiseptic agents. At the time of preparation, methods for preparation may be introduced in accordance with the respective materials to be used.

Hereinafter, the present invention may be explained more specifically with the following examples.

### Example 1

### (In-silico search for proteins having a function of interacting with TERT)

Predicting proteins which interact with TERT, a catalytic subunit of telomerase, was conducted in accordance with the prediction method described in the Patent Reference 1. Specifically, the amino acid sequence of TERT was decomposed into oligopeptides having a pre-determined length in order to search in a database for proteins having the amino acid sequence of each of the oligopeptides, or having homologous amino acid sequences to these amino acid sequences. Then, local alignment was conducted between the proteins obtained and TERT to identify proteins having a high local alignment score that might be capable of interacting with TERT.

As a result of analysis, NEDD8 and USP21 were identified as proteins predicted to have a function of interacting with TERT (Figure 1-A and Figure 1-B, respectively). The amino acid sequence of NEDD8 (SEQ ID NO: 4) was found to contain oligopeptides, ALRGGGG (SEQ ID NO: 9), ALRGGG (SEQ ID NO: 11), VKTLTG (SEQ ID NO: 13) and ILGGSVLHL (SEQ ID NO: 15), which are homologous to oligopeptides consisting of the amino acid resides derived from TERT, ALRGSGA (SEQ ID NO: 8), ARRRGG (SEQ ID NO: 10), VSTLTD (SEQ ID NO: 12) and LLGASVLGL (SEQ ID NO: 14) (Figure 1-A). The amino acid sequence of USP21 (SEQ ID NO: 6) was found to contain oligopeptides, PFAPRARS (SEQ ID NO: 17), RTSGPRPR (SEQ ID NO: 19), SLPIPKK (SEQ ID NO: 21), GRTREP (SEQ ID NO: 23), GRTSGPRPRG (SEQ ID NO: 25), ASEHRL (SEQ ID NO: 27), HRLGRT (SEQ ID NO: 29) and TRPLRD (SEQ ID NO: 31), which are homologous to oligopeptides consisting of the amino acid resides derived from TERT, PRAPRCRA (SEQ ID NO: 16), HASGPRRR (SEQ ID NO: 18), SLPLPKR (SEQ ID NO: 20), GRTRGP (SEQ ID NO: 22), GRTRGPSDRG (SEQ ID NO: 24), AAEHRL (SEQ ID NO: 26), HRAWRT (SEQ ID NO: 28) and TSPLRD (SEQ ID NO: 30) (Figure 1-B).

### Example 2

### (Binding of TERT to USP21 and to NEDD8)

Binding of TERT to both USP21 and NEDD8 was examined in vivo, using a cell co-expression system. The binding of TERT to USP21 was further examined by an *in vitro* binding assay using a pull-down method.

### <materials and preparation thereof>

Human TERT cDNA, human USP21 cDNA and human NEDD8 cDNA were cloned from human thymus cDNA, human kidney cDNA and human brain cDNA (all purchased from Clontech), respectively, by reverse transcription polymerase chain reaction (hereinafter, may be referred to RT-PCR).

A plasmid for expressing TERT in animal cells was constructed by introducing human TERT cDNA into an animal cell expression vector, pCIneo (Promega). The plasmid obtained in this manner is referred to as pCIneo-TERT.

A plasmid for expressing N-terminal FLAG-tagged TERT in animal cells was constructed by introducing human TERT cDNA having a FLAG-tag coding sequence inserted just before the initiation codon into pCI (Clontech). The plasmid obtained in this manner is referred to as pCI-FLAG-TERT.

A plasmid for expressing N-terminal HA-tagged USP21 in animal cells was constructed by introducing human USP21 cDNA having an HA-tag coding sequence inserted just before the initiation codon into pCI (Clontech). The plasmid obtained in this manner is referred to as pCI-HA-USP21.

In addition, a plasmid was constructed for expressing inactive human USP21 having alanine substituting for cysteine at the position of 221 in USP21 that is essential for the cysteine protease activity because the cysteine is an active center of the cysteine protease (Non-patent Reference 9). Specifically, the codon encoding the cysteine in the USP21 gene was substituted with a codon encoding alanine to prepare inactive human USP21 cDNA. Subsequently, the cDNA in which an HA-tag coding sequence was inserted just before the initiation codon was introduced into pCI to construct plasmids for expressing N-terminal HA-tagged inactive USP21 in animal cells. The plasmids obtained in this manner are referred to as pCI-HA-USP21C221A.

Further, a plasmid for expressing N-terminal GST fusion USP21 in E. coli, and a plasmid for expressing N-terminal GST fusion inactive USP21 in E. coli were constructed by introducing human USP21 cDNA and inactive human USP21 cDNA respectively into pGEX-4T (Amersham Biosciences). The plasmids obtained in this manner are referred to as pGEX-USP21 and pGEX -USP21C221A, respectively.

A plasmid for expressing N-terminal HA-tagged NEDD8 in animal cells and a plasmid for expressing N-terminal FLAG-tagged NEDD8 in animal cells were constructed by introducing into pCI (Clontech), human NEDD8 cDNA, in which an HA-tag coding sequence and a FLAG-tag coding sequence were respectively inserted just before the initiation codon. The plasmids obtained in this manner are referred to as pCI-HA-NEDD8 and pCI-FLAG-NEDD8, respectively.

### < in vivo binding assay (a cell co-expression/immunoprecipitation method)>

4 × 10⁵ HEK293T cells were cultured overnight at 37°C under the atmosphere of 5% CO₂ in a dish with 60 mm diameter, and then transfected with 2 µg of pCI-FLAG-TERT alone or in combination with 2 µg of pCI-HA-USP21 or pCI-HA-NEDD8, using FuGENE6 Transfection Reagent (Roche). After 2 days of culturing, the cells were collected and suspended in 500 µl of cell lysis buffer (20 mM Tris-HCl, pH7.4/ 150 mM NaCl/ 1 mM ethylenediaminetetraacetic acid (EDTA)/ 1 mM ethylene glycol-bis-tetraacetic acid (EGTA)/ 1% Triton X-100/ 2.5 mM sodium pyrophsphate/ 1 mM β-glycerophsphate/ 1 mM Na₃VO₄/ protease inhibitor cocktail), and then left to stand on ice for 20 minutes. Subsequently, centrifugation was conducted at 4°C for 10 minutes at 14,000 rpm to collect the supernatants. The supernatants that were collected were used as cell lysates. Next, 450 µl of the cell lysate was added with 10 µl of normal rabbit IgG-agarose (Sigma) that was previously blocked with 0.1% bovine serum albumin (BSA), and then subjected to overturned mixing at 4°C for 1 hour, followed by centrifugation to collect a supernatant. The resulting supernatant was added to 10 µl of anti-HA antibody-conjugated agarose (anti-HA(Y-11)-agaraose, Santa cruz), and then subjected to overturned mixing at 4°C overnight, followed by centrifugation to collect the beads. The beads were washed with the cell lysis buffer four times, and then added with 30 µl of 2 × SDS sample buffer (125 mM Tris HCl, pH 6.8/ 4 % sodium dodecylsulphate (SDS)/ 20 % glycerol/ 0.01 % bromophenol blue). After heating at 100°C for 5 minutes, the supernatant was subjected to separation by 5-20 % SDS-polyacrylamide gel electrophoresis (SDS-PAGE). Then, Western blotting was conducted using anti-FLAG M2 antibodies (Sigma) to detect co-precipitated N-terminal FLAG-tagged TERT (hereinafter, referred to as FLAG-TERT). In addition, Western blotting was conducted using anti-FLAG antibodies (Sigma) and anti-HA antibodies (Y-11, Santa cruz) to determine the amount of TERT and USP21 in the cell lysates, respectively. The detection was conducted using ECL Western blotting detection kit (Amersham Biosciences).

### < in vitro binding assay (a GST pull-down method)>

GST, N-terminal GST fusion USP21 (hereinafter, referred to as GST-USP21) and N-terminal GST fusion inactive USP21 (hereinafter, referred to as GST-USP21C221A) were prepared by transfecting E. coli BL21 strain with pGEX-4T, pGEX-USP21 and pGEX-USP21C221A, respectively, followed by induction of expression and subsequent purification with glutathione sepharose. TERT was prepared by *in vitro* synthesis using TNT^{®} T7 quick coupled transcription/translation system (Promega) and ³⁵S-methionine, where pCIneo-TERT was used as a template. 5 µg of GST, GST-USP21, or GST-USP21C221A was mixed with 20 µl of *in vitro* synthesis reaction solution containing TERT, in binding buffer (20 mM Tris-HCl, pH 8.0/140 mM NaCl/l mM EGTA/l mM dithiothreitol (DTT)/l % TritonX-100) in a final volume of 500µl, and left to stand on ice for 1 hour. After that, 20µl of Glutathione sepharose 4 (Amersham Biosciences) was added thereto, and subjected to overturned mixing at 4°C overnight, followed by centrifugation to collect the beads. The beads were washed with 500µl of the binding buffer four times, and then combined with 20 µl of 2 × SDS sample buffer (125 mM Tris HCl, pH 6.8/ 4 % SDS/ 20 % glycerol/ 0.01 % bromophenol blue). After heating at 100°C for 5 minutes, the supernatant was subjected to separation by 5-20 % SDS-PAGE. Then, TERT was detected by using FLA3000 (Fuji Film). ³⁵S-methionine labeled TERT was uses as a control for TERT detection by SDS-PAGE.

### <results>

When conducting immunoprecipitation of HA-USP21 with anti-HA antibodies, the cell lysate prepared from the cultured cells in which FLAG-TERT was transiently co-expressed with N-terminal HA-tagged USP21 (hereinafter, HA-USP21), showed co-precipitation of protein detected with anti-FLAG antibodies (Figure 2, lane 2, bottom panel). The co-precipitated protein was assumed to be FLAG-TERT. In contrast, such co-precipitation of FLAG-TERT with anti-HA antibodies was not found when using the cells only expressing FLAG-TERT (Figure 2, lane 1, bottom panel). The expression of HA-USP21 or FLAG-TERT in the cultured cells was confirmed using anti-HA antibodies or anti-FLAG antibodies, respectively (Figure 2, lanes 1 and 2, top panel and middle panel). These results suggested that TERT may bind to USP21.

Both GST-USP21 and GST-USP21C221A were found to bind to TERT in the *in vitro* binding assay using a GST pull-down method (Figure 3, lanes 3 and 4, respectively). In contrast, the binding of GST to TERT was not observed (Figure 2, lane 2).

This result revealed that TERT was bound directly to USP21. Further it was revealed that USP21C221A, an inactive USP21, was bound to TERT.

When conducting immunoprecipitation of HA-NEDD8 with anti-HA antibodies, the cell lysate prepared from the cultured cells in which FLAG-TERT was transiently co-expressed with N-terminal HA-tagged NEDD8 (hereinafter, HA-NEDD8), showed co-precipitation of protein detected with anti-FLAG antibodies (Figure 4, lane 2, upper panel). The co-precipitated protein was assumed to be FLAG-TERT. However, signals indicating the co-precipitated protein were detected in a high molecular region compared to a molecular region of FLAG-TERT, which showed smear-like bands. NEDD8 is a ubiquitin-like protein, and is known to covalently conjugate to protein for post-translational modification of proteins. This fact suggested that the signals detected in the high molecular region may be FLAG-TERT modified with one or more HA-NEDD8 molecules. In contrast, the precipitation of FLAG-TERT with anti-HA antibodies and the smear-like signals in a high molecular region were not observed when using the cells only expressing FLAG-TERT (Figure 4, lane 1, upper panel). The amount of FLAG-TERT in the cell lysates used was almost the same for the cells in which FLAG-TERT only was expressed and the cells in which FLAG-TERT was co-expressed with HA-NEDD8 (Figure 4, lanes 1 and 2, respectively, lower panel).

The aforementioned results revealed that TERT was bound to NEDD8. Further, the results suggested that TERT may be bound with one or more NEDD8 molecules resulting in NEDD8 conjugation to TERT.

### Example 3

### (NEDD8 conjugation to TERT and NEDD8 deconjugation by USP21 from NEDD8-conjugated TERT)

Example 2 suggested a possibility of NEDD8 conjugation to TERT. Then, it was examined, using a binding assay employing a cell co-expression/immunocoprecipitation method similar to Example 2, whether NEDD8-conjugated TERT undergoes NEDD8 deconjugation by USP21 that is reported to be a deubiquitination/NEDD8 deconjugation protease (Non-patent Reference 9).

### <materials>

The TERT expression plasmid, the N-terminal FLAG-tagged NEDD8 expression plasmid, the N-terminal HA-tagged USP21 expression plasmid, and the N-terminal HA-tagged USP21C221 A expression plasmid, which were prepared as in Example 2, were used.

### <methods>

4 × 10⁵ HEK293T cells were cultured overnight at 37°C under the atmosphere of 5% CO₂ in a dish with 60 mm diameter, and then transfected with 2 µg of pCIneo-TERT and 2 µg of pCI-FLAG-NEDD8 together with 2 µg of pCI-HA-USP21 or 2 µg ofpCI-HA-USP21C221A in various combinations, using FuGENE6 Transfection Reagent (Roche). After 2 days of culturing, the cells were collected and suspended in 500 µl RIPA buffer (20 mM Tris-HCl, pH7.4/ 150 mM NaCl/ 1 mM EDTA/ 1 mM EGTA/ 1% Triton X-100/ 0.5% sodium deoxycholate/ 0.1% SDS/ 2.5 mM sodium pyrophsphate/ 1 mM β-glycerophsphate/ 1 mM Na₃VO₄/ protease inhibitor cocktail), subjected to sonication for 10 seconds, and then left to stand on ice for 20 minutes. Subsequently, centrifugation was conducted at 4°C for 10 minutes at 14,000 rpm to collect the supernatant. The collected supernatants were used as cell lysates. Next, 450 µl of the cell lysate was combined with 15 µl of protein G-Sepharose (Amersham Biosciences) that was previously blocked with 0.1 % BSA, and then subjected to overturned mixing at 4°C for 1 hour, followed by centrifugation to collect a supernatant. The collected supernatant was combined with 2 µg of anti-TERT (L-21) antibodies (Santa cruz), and then subjected to overturned mixing at 4°C for four hours. After that, 15 µl of protein-G Sepharose was added thereto and subjected to overturned mixing at 4°C overnight, followed by centrifugation to collect the beads. The beads were washed with 500 µl of the RIPA buffer four times, and then combined with 30 µl of 2 × SDS sample buffer. After heating at 100°C for 5 minutes, the supernatant was subjected to separation by 5-20 % SDS-PAGE. Then, Western blotting was conducted using anti-FLAG M2 antibodies (Sigma) to detect TERT modified with N-terminal FLAG-tagged NEDD8 (hereinafter, FLAG- NEDD8). In addition, Western blotting was conducted using anti-TERT (L-20) antibodies (Sigma) and anti-HA (Y11) antibodies (both purchased from Santa Cruz) to determine the amount of TERT, USP21, and the inactive USP21 in the cell lysates, respectively. The detection was conducted using an ECL Western blotting detection kit (Amersham Biosciences).

### <results>

The cell lysate prepared from the cultured cells in which TERT was transiently co-expressed with FLAG-NEDD8 showed smear-like signals detected in a high molecular region which was assumed to be TERT modified with one or more FLAG-NEDD8 molecules (Figure 5, lane 3, top panel). Such signals were not detected when using the cell lysates prepared from the cultured cells in which only TERT or only FLAG-NEDD8 was expressed (Figure 5, lanes 1 and 2, respectively, top panel). These results revealed the NEDD8 conjugation to TERT.

The smear-like signals detected in a high molecular region as above, namely signals indicating TERT modified with one or more FLAG-NEDD8 molecules, were significantly decreased by co-expression of HA-USP21 (Figure 5, lane 4, top panel). Such decrease of the signals was not observed when co-expressing the inactive USP21, HA-USP21C221A, instead of HA-USP21 (Figure 5, lane 5, top panel). The amount of TERT in the cell lysates used was almost the same for the cells in which only TERT was expressed, the cells in which TERT was co-expressed with FLAG-NEDD8, and the cells in which TERT and FLAG-NEDD8 were co-expressed together with HA-UAP21 or HA-UAP21 C221 A (Figure 5, lanes 1, 3, 4 and 5, respectively, middle panel). Further, the expression of HA-UAP21 or HA-UAP21C221A in the cell lysates used was confirmed (Figure 5, lanes 4 and 5, respectively, bottom panel).

The aforementioned results revealed that NEDD8 deconjugation from NEDD8-conjugated TERT was caused by USP21. Further, it was revealed that NEDD8 deconjugation from NEDD8-conjugated TERT was not caused by the inactive USP21, USP21C221A.

### Example 4

### (proteasome-dependent degradation of TERT)

NEDD8 is a ubiquitin-like protein, and is known to covalently conjugate to proteins for post translational modification of proteins. Only a cullin family protein, which is one of the subunits of a ubiquitin ligase complex, has been reported as a protein that undergoes NEDD8 conjugation (Non-patent References 5 and 6). In addition, it has been suggested that there exists a mechanism of regulation of ubiquitin-ligase activity through NEDD8 conjugation to cullin. Also, as ubiqitinated proteins are known to undergo proteasome-dependent degradation, it has been suggested that ubiquitination is involved in regulation of protein stability (Non-patent Reference 5). NEDD8 has a significant homology with ubiquitin at the level of both primary structure and tertiary structure. Therefore, it is plausible that NEDD8 conjugation to proteins works as a signal for proteasome-dependent protein degradation (Non-patent Reference 5), similar to ubiquitination. Thus, it can be reasoned, that there exists a regulatory system for TERT degradation via modification by NEDD8 conjugation to TERT and NEDD8 deconjugation by USP21. Accordingly, an investigation was conducted to determine whether TERT may undergo proteasome-dependent degradation. Specifically, the effect of a proteasome inhibitor, MG132, on the amount of intracellular TERT was studied using a transient expression system for TERT in HeLa cells, as described hereunder.

### <materials>

The TERT expression plasmid which was prepared as in Example 2 was used.

### <methods>

8 × 10⁴ cells/well of HEK293T cells were cultured overnight at 37°C under the atmosphere of 5% CO₂ in 12 well plates, and then transfected with 1 µg of pCIneo-TERT using FuGENE6 Transfection Reagent (Roche). After 48 hours of culturing, the culture was added with proteasome inhibitor, MG-132 (Calbiochem) to a final concentration of 25 µM, followed by further culturing for 24 hours. After that, the cells were collected. As a control, cells were prepared for use by adding the same amount of dimethyl sulphoxide (DMSO) in culture instead of adding MG-132 and culturing as similar to the above. The collected cells were suspended in 60 µl of the RIPA buffer (same composition as that of the RIPA buffer described in Example 3), subjected to sonication for 10 seconds, and then left to stand on ice for 20 minutes. Subsequently, centrifugation was conducted at 4°C for 10 minutes at 14,000 rpm to collect the supernatant. The collected supernatants were used as cell lysates. Next, the cell lysates were subjected to separation by 5-20 % SDS-PAGE followed by detection of TERT by Western blotting using anti-TERT (L-20) antibodies (Santa Cruz). Further, β-tubulin, an internal control protein in the cell lysates, was detected by Western blotting using anti-β-tubulin (H-235) antibodies (Santa Cruz). The detection was conducted using an ECL Western blotting detection kit (Amersham Biosciences).

### <results>

The cell lysate prepared from the cells treated with MG132 showed increased amount of TERT (Figure 6, lane 2, upper panel) in comparison to the cell lysate prepared from the cells not treated with MG132 (Figure 6, lane 1, upper panel). There was no difference found in the amount of β-tubulin, an internal protein (Figure 6, lanes 1 and 2, lower panel). These results suggested that treatment of the proteasome inhibitor, MG132, led to inhibition of proteasome-dependent degradation of TERT. Thus, TERT was found to undergo proteasome-dependent degradation.

### Example 5

### (USP21 induced stabilization of TERT)

Whether NEDD8 deconjugation by USP21 from NEDD8-conjugated TERT may lead to inhibition of proteasome-dependent degradation of TERT was examined. Such an examination reveals a possibility of stabilization of TERT by USP21. Specifically, the effect of transient overexpression of USP21 on the amount of intracellular TERT was studied using a transient expression system for TERT in HEK293T cells, as described hereunder.

### <materials>

The TERT expression plasmid, the N-terminal HA-tagged USP21 expression plasmid and the N-terminal HA-tagged USP21C221A expression plasmid which were prepared as in Example 2 were used.

### <methods>

2 × 10⁵ cells/well of HEK293T cells were cultured overnight at 37°C under the atmosphere of 5% CO₂ in 6 well plates, and then transfected with 1 µg of pCIneo-TERT together with 1 µg of pCI (empty vector), pCI-HA-UPA21 or pCI-HA-USP21C221A, using FuGENE6 Transfection Reagent (Roche). After 2 days of culturing, the cells were collected and suspended in 200 µl of the RIPA buffer (same composition as that of the RIPA buffer described in Example 3), subjected to sonication for 10 seconds, and then left to stand on ice for 20 minutes. Subsequently, centrifugation was conducted at 4°C for 10 minutes at 14,000 rpm to collect the supernatant. The collected supernatants were used as cell lysates. Next, the cell lysates were subjected to separation by 5-20 % SDS-PAGE followed by detection of TERT and HA-USP21 by Western blotting using anti-TERT (L-20) antibodies (Santa Cruz) and anti-HA (Y-11) antibodies (Santa Cruz), respectively. Further, β-tubulin, an internal control protein in the cell lysates, was detected by Western blotting using anti-β-tubulin (H-235) antibodies (Santa Cruz). The detection was conducted using an ECL Western blotting detection kit (Amersham Biosciences).

### <results>

The cell lysate prepared from the cells in which HA-USP21 was co-expressed with TERT showed an increased amount of TERT (Figure 7, lane 3, top panel) in comparison to the cell lysate prepared from the cells in which TERT only was expressed or TERT was expressed together with USP21C221A (Figure 7, lanes 2 and 4, respectively, top panel). This result suggested that a transient overexpression of USP21 may lead to stabilization of TERT. There was no difference found in the amount of β-tubulin, an internal protein (Figure 7, lanes 1 to 4, bottom panel). Therefore, it is evident that the increased amount of TERT, as described above, did not result from the difference between the amount of protein in the samples. The amount of USP21 and that of USP21 C221 A in the cell lysates used was almost the same (Figure 7, lanes 3 and 4, middle panel).

The above results revealed that NEDD8 deconjugation by USP21 from NEDD8-conjugated TERT led to inhibition of proteasome-dependent degradation of TERT.

### Example 6

### (USP21 induced increase of exogenous TERT-dependent intracellular telomerase activity)

The results obtained in Examples 2 to 5 strongly suggested that there may exist a regulatory mechanism of TERT degradation via NEDD8 conjugation to TERT and NEDD8 deconjugation by USP21 from NEDD8-conjugated TERT. Since TERT is a catalytic subunit of telomerase, the inventors reasoned that such a regulatory mechanism of TERT degradation may be involved in regulation of telomerase activity. Then, an investigation was conducted to determine whether USP21 is actually involved in regulation of telomerase activity. Specifically, the effect of USP21 overexpression on the exogenous TERT expression-dependent telomerase activity was studied using a transient expression system of TERT in SaOS2 cells (human osteosarcoma cells) having no native telomerase activity, as described hereunder. As SaOS2 cells express TR gene only, and not TERT gene, they do not have telomerase activity.

### <materials>

The TERT expression plasmid, the N-terminal HA-tagged USP21 expression plasmid and the N-terminal HA-tagged USP21C221A expression plasmid which were prepared as in Example 2 were used.

### <methods>

4 × 10⁵ cells/well of SaOS2 cells were cultured overnight at 37°C under the atmosphere of 5% CO₂ in 6 well plates, and then transfected with 0.8 µg of pCIneo-TERT together with 2 µg of pCI (empty vector), pCI-HA-UPA21 or pCI-HA-USP21C221A, using Lipofectamine 2000 (Invitrogen). After 24 hours of culturing, the cells were collected to prepare nuclear extracts using NE-PER Nuclear and Cytoplasmic Extraction Reagents (Pierce). The preparation was conducted in accordance with the attached instruction manuals. After measuring the protein concentration in the nuclear extracts by using Coomassie Plus-200 Protein Assay Reagent (Pierce), the nuclear extracts (protein concentration, 20 ng) were used for TRAP reaction. TRAP reaction was conducted using TRAPEZE XL Kit (Intergen) in accordance with the attached instruction manuals. Specifically, after carrying out telomere elongation reaction at 30°C for 30 minutes, the sequence of generated telomere repeats was amplified by PCR. PCR cycle conditions were 36 cycles consisting of a 30 seconds reaction at 94°C, a 30 second reaction at 59°C and a 1 minute reaction at 72°C, and then finally a 25 minute reaction at 55°C. After carrying out PCR, the fluorescent intensity of the reaction solutions was determined with a fluorescence spectrophotometer, F-2000 (Hitachi) to caluculate telomerase activity of the nuclear extracts. Specifically, the measurement of the fluorescent intensity was conducted by measuring both fluorescent intensity of fluorescein conjugated to primers used in PCR (index for telomerase reaction products) and sulphorhodamine (index for internal standard) with respective wave lengths (1 Ex = 485 nm/ 1Em = 535 nm and 1 Ex = 585 nm/ 1Em = 620 nm, respectively). Telomerase activity was exhibited by a ratio to the internal standard (ΔFL/ΔRL). The amount of TERT and HA-USP21 in the prepared nuclear extracts was determined by Western blotting using anti-TERT (L-20) antibodies (Santa Cruz) and anti-HA (Y-11) antibodies (Santa Cruz), respectively.

### <results>

The expression of TERT gene allows SaOS2 cells, which have low nuclear telomerase activity as only TR gene and not TERT gene is expressed, to show increased telomerase activity (Figure 8-A, columns 2 to 4). Further, the overexpression of TERT together with N-terminal HA-tagged USP21 (HA-USP21) in SaOS2 cells resulted in increased nuclear telomerase activity in comparison to expression of TERT only (Figure 8-A, column 3). Such an increase in nuclear telomerase activity was not observed by overexpression of the inactive USP21, HA-USP21 C221 A (Figure 8-A, column 4). From these results, it can be considered that the increased nuclear telomerase activity caused by overexpression of HA-USP21 depends on cysteine protease activity of USP21. Further, as a result of Western blotting for measuring the amount of TERT in the nuclear extract prepared from the SaOS2 cells in which TERT was transiently expressed, the nuclear extract prepared from the cells in which HA-USP21 was overexpressed showed an increased amount of TERT in comparison to the nuclear extract prepared from the cells in which HA-USP21 was not expressed (Figure 8-B, lane 2, upper panel). The nuclear extract prepared from the cells in which HA-USP21C221A was overexpressed did not show an increase in the amount of TERT (Figure 8-B, lane 3, upper panel). Further, the expression of HA-USP21 and HA-USP21C221A in the cells were confirmed (Figure 8-B, lanes 2 and 3, respectively, lower panel).

These results suggested that the increased telomerase activity caused by overexpression of USP21 was dependent on an increase of TERT, which corresponds to the results in the Example 5 showing stabilization of TERT by USP21. Thus, it can be considered that stabilization of TERT by USP21 led to increased nuclear telomerase activity.

### Example 7

### (USP21 induced increase of native telomerase activity in cells)

The effect of overexpression of USP21 on native telomerase activity was examined using HEK293T cells having native telomerase activity.

### <materials>

The N-terminal HA-tagged USP21 expression plasmid and the N-terminal HA-tagged USP21 C221 A expression plasmid which were prepared as in Example 2 were used.

### <methods>

1 × 10⁵ cells/well of HEK293T cells were cultured overnight at 37°C under the atmosphere of 5% CO₂ in 6 well plates, and then transfected with 1 µg of pCI (empty vector), pCI-HA-UPA21 or pCI-HA-USP21C221A using FuGENE6 Transfection Reagent (Roche). After 48 hours of culturing, the cells were collected to prepare nuclear extracts using NE-PER Nuclear and Cytoplasmic Extraction Reagents (Pierce). The preparation was conducted in accordance with the attached instruction manuals. After measuring the protein concentration in the nuclear extracts by using Coomassie Plus-200 Protein Assay Reagent (Pierce), the nuclear extracts (protein concentration, 10 ng) were used for TRAP reaction to determine telomerase activity. TRAP reaction and calculation of telomerase activity were conducted using the same method as described in Example 6.

### <results>

The overexpression of HA-USP21 led to an increase of nuclear telomerase activity (Figure 9, column 2). The overexpression of inactive USP21, HA-USP21C221A, did not lead to an increase of telomerase activity (Figure 9, column 3). These results revealed that the increase of telomerase activity caused by overexpression of HA-USP21 was dependent on cysteine protease activity of USP21 (Figure 9). Thus, it was found that USP21 increased nuclear telomerase activity in its cysteine protease activity dependent manner.

### Example 8

### (expression of USP21 in cancer cell lines)

Whether USP21 may be expressed in HeLa cells, a cell line originated in human uterus cancer was examined by RT-PCR.

### <methods>

Total RNA was prepared from HeLa cells and used for detection of USP21 mRNA by RT-PCR. Specifically, total RNA was prepared from HeLa cells using RNeasy Mini Kit (Qiagen), and then used for synthesis of HeLa cell-derived cDNA using Omniscript reverse transcriptase (Qiagen). The HeLa cDNA prepared in this manner was used as a template for conducting PCR using HotStarTaq DNA polymerase (Qiagen) and human USP21 gene-specific primers. The reaction solution was subjected to electrophoresis with 2.5% agarose gel, followed by detection of a PCR product of interest by ethidiumbromide staining. With regard to human USP21 gene-specific primers, two primer pairs were used, one of which is a pair of the forward primer 1 (1F, SEQ ID NO: 32) and the reverse primer 1 (1R, SEQ ID NO: 33), and the other is a pair of the forward primer 2 (2F, SEQ ID NO: 34) and the reverse primer 2 (1R, SEQ ID NO: 35). Both primer pairs were designed so as to allow amplification of 101 bp fragments of USP21 cDNA.

### <results>

The examination of the expression of USP21 in HeLa cells by RT-PCR using the two primer pairs showed that both primer pairs gave amplification products with an expected size of 101bp (Figure 10, lanes 2 and 3). The human USP21 gene-specific primers used herein were designed so as to be intron-spanning primers, so that the amplified PCR products are not genomic DNA-derived products.

The results as described above strongly suggest that there may exist a regulatory system for TERT degradation via NEDD8 conjugation to TERT and NEDD8 deconjugation by USP21 from NEDD8-conjugated TERT, and that the regulatory system for TERT degradation can regulate telomerase activity in cells.

### INDUSTRIAL APPLICABILITY

The present invention can provide a method of identifying a compound that inhibits telomerase activity. For example, a method of identifying a compound that inhibits binding of USP21 to NEDD8-conjugated TERT, a method of identifying a compound that inhibits NEDD8 deconjugation by USP21 from NEDD8-conjugated TERT, and the like, can be provided.

In addition, the present invention provides enablement for inhibiting telomerase activity. Further, the present invention provides enablement for prevention and/or treatment of diseases attributable to increased telomerase activity. Since telomerase is expressed in most cancer cells regardless of the kinds of cancer and is not expressed in normal somatic cells except for germ cells, the present invention is assumed to be effective for prevention and/or treatment of various cancer diseases.

Thus, the present invention is useful for basic research and the like on telomerase-related cell growth and cell aging mechanism, telomerase function, and diseases attributable to increased telomerase activity. Further, the present invention is useful for development of medicaments and treatment methods applicable for diseases attributable to increased telomerase activity such as cancer diseases.

### SEQUENCE LISTING FREE TEXT

**[0189]** SEQ ID NO: 1: human TERT gene
SEQ ID NO: 2: A protein encoded by human TERT gene (SEQ ID NO: 1)
SEQ ID NO: 3: human NEDD8 gene
SEQ ID NO: 4: A protein encoded by human NEDD8 gene (SEQ ID NO: 3)
SEQ ID NO: 5: human USP21 gene
SEQ ID NO: 6: A protein encoded by human USP21 gene (SEQ ID NO: 5)
SEQ ID NO: 7: A designed polypeptide having a substitution mutation at the position of 221 in the amino acid sequence of SEQ ID NO: 6 from cystein to alanine
SEQ ID NO: 8: A partial sequence of TERT (SEQ ID NO: 2), which is highly homologous to a partial sequence (SEQ ID NO: 9) of NEDD8
SEQ ID NO: 9: A partial sequence of NEDD8 (SEQ ID NO: 4), which is highly homologous to a partial sequence (SEQ ID NO: 8) of TERT
SEQ ID NO: 10: A partial sequence of TERT (SEQ ID NO: 2), which is highly homologous to a partial sequence (SEQ ID NO: 11) of NEDD8
SEQ ID NO: 11: A partial sequence of NEDD8 (SEQ ID NO: 4), which is highly homologous to a partial sequence (SEQ ID NO: 10) of TERT
SEQ ID NO: 12: A partial sequence of TERT (SEQ ID NO: 2), which is highly homologous to a partial sequence (SEQ ID NO: 13) of NEDD8
SEQ ID NO: 13: A partial sequence of NEDD8 (SEQ ID NO: 4), which is highly homologous to a partial sequence (SEQ ID NO: 12) of TERT
SEQ ID NO: 14: A partial sequence of TERT (SEQ ID NO: 2), which is highly homologous to a partial sequence (SEQ ID NO: 15) of NEDD8
SEQ ID NO: 15: A partial sequence of NEDD8 (SEQ ID NO: 4), which is highly homologous to a partial sequence (SEQ ID NO: 14) of TERT
SEQ ID NO: 16: A partial sequence of TERT (SEQ ID NO: 2), which is highly homologous to a partial sequence (SEQ ID NO: 17) of USP21
SEQ ID NO: 17: A partial sequence of USP21 (SEQ ID NO: 6), which is highly homologous to a partial sequence (SEQ ID NO: 16) of TERT
SEQ ID NO: 18: A partial sequence of TERT (SEQ ID NO: 2), which is highly homologous to a partial sequence (SEQ ID NO: 19) of USP21
SEQ ID NO: 19: A partial sequence of USP21 (SEQ ID NO: 6), which is highly homologous to a partial sequence (SEQ ID NO: 18) of TERT
SEQ ID NO: 20: A partial sequence of TERT (SEQ ID NO: 2), which is highly homologous to a partial sequence (SEQ ID NO: 21) of USP21
SEQ ID NO: 21: A partial sequence of USP21 (SEQ ID NO: 6), which is highly homologous to a partial sequence (SEQ ID NO: 20) of TERT
SEQ ID NO: 22: A partial sequence of TERT (SEQ ID NO: 2), which is highly homologous to a partial sequence (SEQ ID NO: 23) of USP21
SEQ ID NO: 23: A partial sequence of USP21 (SEQ ID NO: 6), which is highly homologous to a partial sequence (SEQ ID NO: 22) of TERT
SEQ ID NO: 24: A partial sequence of TERT (SEQ ID NO: 2), which is highly homologous to a partial sequence (SEQ ID NO: 25) of USP21
SEQ ID NO: 25: A partial sequence of USP21 (SEQ ID NO: 6), which is highly homologous to a partial sequence (SEQ ID NO: 24) of TERT
SEQ ID NO: 26: A partial sequence of TERT (SEQ ID NO: 2), which is highly homologous to a partial sequence (SEQ ID NO: 27) of USP21
SEQ ID NO: 27: A partial sequence of USP21 (SEQ ID NO: 6), which is highly homologous to a partial sequence (SEQ ID NO: 26) of TERT
SEQ ID NO: 28: A partial sequence of TERT (SEQ ID NO: 2), which is highly homologous to a partial sequence (SEQ ID NO: 29) of USP21
SEQ ID NO: 29: A partial sequence of USP21 (SEQ ID NO: 6), which is highly homologous to a partial sequence (SEQ ID NO: 28) of TERT
SEQ ID NO: 30: A partial sequence of TERT (SEQ ID NO: 2), which is highly homologous to a partial sequence (SEQ ID NO: 31) of USP21
SEQ ID NO: 31: A partial sequence of USP21 (SEQ ID NO: 6), which is highly homologous to a partial sequence (SEQ ID NO: 30) of TERT
SEQ ID NO: 32: A forward primer designed for use in PCR to specifically amplify a fragment of USP21 cDNA
SEQ ID NO: 33: A reverse primer designed for use in PCR to specifically amplify a fragment of USP21 cDNA
SEQ ID NO: 34: A forward primer designed for use in PCR to specifically amplify a fragment of USP21 cDNA
SEQ ID NO: 35: A reverse primer designed for use in PCR to specifically amplify a fragment of USP21 cDNA

## Claims

1. A method of enhancing degradation of TERT, comprising inhibiting binding of NEDD8-conjugated TERT to USP21.

2. A method of enhancing degradation of TERT, comprising inhibiting NEDD8 deconjugation by USP21 from NEDD8-conjugated TERT.

3. A method of inhibiting telomerase activity, comprising utilizing the method of enhancing degradation of TERT according to claim 1 or claim 2.

4. A method of identifying a compound that inhibits binding of USP21 to TERT, comprising: contacting a compound to USP21 and/or TERT under conditions where the interaction of the compound with USP21 and/or TERT are allowed; introducing a system using a signal and/or a marker that is generated by the binding of USP21 to TERT; and detecting the presence, absence or change of the signal and/or the marker to determine whether the compound inhibits the binding of USP21 to TERT.

5. A method of identifying a compound that inhibits binding of USP21 to NEDD8-conjugated TERT, comprising: contacting a compound to USP21 and/or NEDD8-conjugated TERT under conditions where the interaction of the compound with USP21 and/or NEDD8-conjugated TERT are allowed; introducing a system using a signal and/or a marker that is generated by the binding of USP21 to NEDD8-conjugated TERT; and detecting the presence, absence or change of the signal and/or the marker to determine whether the compound inhibits the binding of USP21 to NEDD8-conjugated TERT.

6. A method of identifying a compound that inhibits NEDD8 deconjugation by USP21 from NEDD8-conjugated TERT, comprising: contacting a compound to USP21 and/or NEDD8-conjugated TERT under conditions where the interaction of the compound with USP21 and/or NEDD8-conjugated TERT are allowed; introducing a system using a signal and/or a marker that is generated by the NEDD8 deconjugation by USP21 from NEDD8-conjugated TERT; and detecting the presence, absence or change of the signal and/or the marker to determine whether the compound inhibits the NEDD8 deconjugation by USP21 from NEDD8-conjugated TERT.

7. An agent for inhibiting telomerase activity, containing a compound that inhibits binding of USP21 to NEDD8-conjugated TERT.

8. An agent for inhibiting telomerase activity, containing a compound that inhibits NEDD8 deconjugation by USP21 from NEDD8-conjugated TERT.

9. An agent for preventing and/or treating diseases attributable to increased telomerase activity, containing the agent for inhibiting telomerase activity according to claim 7 or claim 8.

10. An anti-cancer agent, containing the agent for inhibiting telomerase activity according to claim 7 or claim 8.

11. A method of preventing and/or treating diseases attributable to increased telomerase activity, comprising utilizing the method of inhibiting telomerase activity according to claim 3.

12. A method of preventing and/or treating cancer diseases, comprising utilizing the method of inhibiting telomerase activity according to claim 3.

13. A method of preventing and/or treating diseases attributable to increased telomerase activity, comprising utilizing the agent for inhibiting telomerase activity according to claim 7 or claim 8.

14. A method of preventing and/or treating cancer diseases, comprising utilizing the agent for inhibiting telomerase activity according to claim 7 or claim 8.

15. A reagent kit, comprising at least one member selected from the group consisting of USP21, a polynucleotide encoding USP21, a vector containing the polynucleotide, and a transformant containing the vector; at least one member selected from the group consisting of TERT, a polynucleotide encoding TERT, a vector containing the polynucleotide, and a transformant containing the vector; and at least one member selected from the group consisting of NEDD8, a polynucleotide encoding NEDD8, a vector containing the polynucleotide, and a transformant containing the vector.
